(19) Europäisches Patentamt

European Patent Office

Office européen des brevets

(11) Veröffentlichungsnummer: **0 626 449 A2**

## (12) EUROPÄISCHE PATENTANMELDUNG

(21) Anmeldenummer: **94107546.7**

(22) Anmeldetag: **16.05.94**

(51) Int. Cl.5: **C12N 15/40**, C12N 15/82, C12N 5/04, A01H 5/00

(30) Priorität: **28.05.93 DE 4317845**

(43) Veröffentlichungstag der Anmeldung:
**30.11.94 Patentblatt 94/48**

(84) Benannte Vertragsstaaten:
**BE CH DE DK ES FR GB IT LI NL SE**

(71) Anmelder: **BAYER AG**

**D-51368 Leverkusen (DE)**

(72) Erfinder: **Schreier, Peter Helmut, Dr.**
**Dasselstrasse 16**
**D-50674 Köln (DE)**
Erfinder: **Stenzel, Klaus, Dr.**
**Seesenerstrasse 17**
**D-40595 Düsseldorf (DE)**
Erfinder: **Adam, Günter, Prof. Dr.**
**Wiedebeinstrasse 23**
**D-38118 Braunschweig (DE)**
Erfinder: **Maiss, Edgar, Dr.**
**Nietschestrasse 26**
**D-38126 Braunschweig (DE)**

(54) **Desoxyribonukleinsäuren, die Pflanzenvirus Sequenzen kodieren.**

(57) Die vorliegende Erfindung betrifft neue, rekombinante Desoxyribonukleinsäuren (DNA), Vektoren und Wirtsorganismen, die diese enthalten sowie transgene Pflanzen, die die rekombinante DNA enthalten und eine erhöhte Resistenz gegen Schadorganismen und Pflanzenkrankheiten aufweisen, wobei die rekombinanten Desoxyribonukleinsäuren (DNA), dadurch gekennzeichnet sind, daß sie aus einer Kombination der folgenden Komponenten oder Komponenten mit einer jeweils gleichwirkenden DNA bestehen oder diese enthalten:
(a) einem doppelsträngigen cDNA Fragment, das von der RNA des Scharkavirus (Plumpoxvirus (PPV)) abgeleitet ist ("Fragment A") und
(b) aus einem doppelsträngigen cDNA Fragment, das von der S RNA des Tomatenbronzefleckenvirus (tomato spotted wilt virus, TSWV) abgeleitet ist ("Fragment B"), wobei diese Kombination, neben gegebenenfalls vorhandenen weiteren DNA Fragmenten, auch einen in Pflanzenzellen wirkenden Promotor aufweisen kann.

Die vorliegende Erfindung betrifft neue, rekombinante Desoxyribonukleinsäuren (DNA), Vektoren und Wirtsorganismen, die diese enthalten sowie transgene Pflanzen, die die rekombinante DNA enthalten und eine erhöhte Resistenz gegen Schadorganismen und Pflanzenkrankheiten aufweisen.

Für eine Reihe von Viren wurde bereits gezeigt, daß sowohl Struktur- als auch Nichtstrukturgene, die aus diesen Viren isoliert und in geeigneter Weise in transgenen Pflanzen zur Expression gebracht wurden, dort zur erhöhten Resistenz gegenüber dem Befall durch das homologe Virus bzw. zur Abschwächung und/oder Verzögerung der Symptomausprägung führten. Zuerst wurde dies am Beispiel des Hüllproteins des Tabak Mosaik Virus (TMV) gezeigt (EP-A2-0 223 452; Beachy et al., 1985; Powell Abel et al., 1986). Das TMV gehört zum Genus Tobamovirus, hat eine vergleichsweise einfache Architektur und besitzt, wie viele andere Pflanzenviren als genetische Information ein einzelsträngiges RNA Molekül mit Plusstranginformation ("single stranded positive sense RNA genome"). Die genetische Information ist in einer Hülle, die aus vielen identischen Untereinheiten besteht, verpackt. Später konnte gezeigt werden, daß eine vergleichbare Strategie, auch für das essentiell verschiedene Tomatenbronzefleckenvirus (Tomato spotted wilt virus, TSWV) im Genus Tospovirus der Familie der Bunyaviridae erfolgreich war (EP-A1-0 426 195; Gielen et al., 1991; De Haan et al., 1992). Der Genus Tospovirus ist charakterisiert als morphologisch spharisches Partikel von ca. 100 nm Durchmesser und ein dreiteiliges, einzelsträngiges RNA-Genom welches als Nukleoproteinkomplex ("nucleocapsid") von einer Lipidhülle umgeben wird. Diese Lipidhülle ist mit Glykoproteinen besetzt.

Das dreiteilige Genom besitzt Ambisense oder Minusstranginformation ("single stranded RNA molecules of negative or ambisense polarity", De Haan et al., 1990). Die Resistenz in transgenen Pflanzen beruht hier auf der Übertragung und Expression des N-Strukturgens eines Tospovirus, dem Tomatenbronzefleckenvirus ("tomato spotted wilt virus" = TSWV). Das N-Genprodukt ist Bestandteil des oben erwähnten Nukleoproteinkomplexes.

Während im Falle des Hüllproteins von TMV eine deutlich ausgeprägte Kreuzresistenz gegenüber nahe verwandten Tobamoviren gezeigt werden konnte (z.B. Tumer et al., 1987), war im Fall von TSWV und der Expression des N-Gens eine solche Kreuzresistenz bisher nicht bekannt (De Haan et al., 1992; MacKenzie and Ellis, 1992; Sheng-Zi et al. 1992).

Es wurden neue rekombinante Desoxyribonukleinsäuren (DNA) gefunden, die dadurch gekennzeichnet sind, daß sie aus einer Kombination der folgenden Komponenten oder Komponenten mit einer jeweils gleichwirkenden DNA bestehen oder diese enthalten:

(a) einem doppelsträngigen cDNA Fragment, das von der RNA des Scharkavirus (Plumpoxvirus (PPV)) abgeleitet ist ("Fragment A") und

(b) aus einem doppelsträngigen cDNA Fragment, das von der S RNA des Tomatenbronzefleckenvirus (tomato spotted wilt virus, TSWV) abgeleitet ist ("Fragment B"), wobei diese Kombination, neben gegebenenfalls vorhandenen weiteren DNA Fragmenten, auch einen in Pflanzenzellen wirkenden Promotor aufweisen kann.

Weiterhin wurde gefunden, daß transgene Pflanzen, die in ihrem Genom die neue rekombinante DNA integriert haben (und diese zur Expression bringen), eine erhöhte Resistenz gegenüber Schadorganismen und Pflanzenkrankheiten aufweisen.

Die transgenen Pflanzen zeigen insbesondere auch eine erhöhte Resistenz nicht nur gegenüber dem homologen TSW Virus-Isolat, aus dem Fragment B abgeleitet wurde, sondern auch gegenüber anderen Tospovirus Species. Dies ist überraschend, da die nach dem Stand der Technik bekannten rekombinanten Desoxyribonukleinsäuren, die von natürlich vorkommenden viralen Genen abgeleitet wurden, keine solche breite Wirkung in den entsprechenden transgenen Pflanzen haben (De Haan et al., 1992; MacKenzie and Ellis, 1992). Besonders überraschend ist darüber hinaus eine erhöhte Resistenz gegenüber Viren zu beobachten, die nicht zum Genus Tospovirus gehören.

In der erfindungsgemäßen rekombinanten DNA liegt das Fragment B hinter (also in 3'-Richtung) dem Fragment A, wobei die beiden Fragmente direkt oder durch ein weiteres DNA Fragment ("Fragment C") miteinander verbunden sind. Falls die erfindungsgemäße DNA eine vollständige Transkriptionseinheit darstellt, ist dem Fragment A ein in Pflanzen wirkender Promotor vorgeschaltet. Weiterhin folgt in diesem Fall dem Fragment B eine 3'-untranslatierte DNA, die direkt oder über ein weiteres DNA Fragment ("Fragment D") mit dem Fragment B verbunden ist. Die 3'-untranslatierte DNA, enthält eine Transkriptionsterminationssequenz (TTS) und eine Polyadenylierungsstelle.

Der Begriff "Transkriptionsterminationssequenz" bedeutet, daß es sich um eine Sequenz handelt, die das Ende der RNA-Synthese von einer bestimmten als Matrize dienenden DNA-Sequenz durch eine bestimmte Polymerase definiert. Im Falle der bei transgenen Pflanzen eingesetzten "chimären Gene" können z.B. die TTS des Nopalin-Synthasegens (Zambryski et al., 1983), des Octopin-Synthasegens (Herrera-Estrella et al., 1983) oder des 35S-Transkripts des CaMV (Cauliflower Mosaic Virus) (Pietrzak et al.,

2

1986) verwendet werden.

Ferner bedeutet der Begriff "Polyadenylierungsstelle" bzw. "Polyadenylierungssignal" vorzugsweise eine aus sechs Nukleotiden (z.B. AAUAAA) bestehende Sequenz, die die exakte Prozessierung des Primärtranskriptes und die posttranskriptionelle Synthese einer "Poly A-Sequenz" an das 3'-Ende des Primärtranskriptes bewerkstelligt.

Hierbei ergibt sich demgemäß für eine vollständige Transkriptionseinheit die folgende DNA-Anordnung: (5')Promotor - Fragment A - gegebenenfalls: Fragment C -Fragment B - gegebenenfalls:Fragment D - 3'- untranslatierte DNA (3')

Die so angeordnete DNA ist Teil der vorliegenden Erfindung. Ebenfalls Bestandteil der vorliegenden Erfindung ist die rekombinante DNA in folgenden Anordnungen:

(5')Fragment A - gegebenenfalls: Fragment C - Fragment B - gegebenenfalls: Fragment D - 3' untranslatierte DNA (3') und

Fragment A - gegebenenfalls. Fragment C - Fragment B - gegebenenfalls: Fragment D (3') und

(5') Promotor - Fragment A- gegebenenfalls: Fragment C - Fragment B (3')

Fragment A ist eine doppelsträngige cDNA, die von der RNA des Scharkavirus (Plumpoxvirus (PPV)) abgeleitet ist, wobei der Begriff "abgeleitet" bedeutet, daß die cDNA eine DNA ist, die komplementär zur Virus RNA vorliegt.

Vorzugsweise wird als Fragment A eine DNA verwendet, wie sie in SEQ ID No:7 wiedergegeben ist.

Fragment B ist eine doppelsträngige cDNA, die von der S RNA und zwar vorzugsweise vom N-Strukturgen des Tomatenbronzefleckenvirus (tomato spotted wilt virus (TSWV)), vorzugsweise des L3 Isolates, abgeleitet ist, wobei der Begriff "abgeleitet" bedeutet, daß die cDNA eine DNA ist, die komplementär zu Virus RNA vorliegt. Vorzugsweise wird als Fragment B eine DNA verwendet, wie sie in SEQ ID No:11 wiedergegeben ist.

Die Fragmente A und B können direkt oder vorzugsweise durch ein doppelsträngiges DNA-Fragment C miteinander verbunden sein. Als Fragment C kann ein synthetisches DNA Fragment verwendet werden, welches so gewählt wird, daß es in einem bestimmten Leseraster kein Stopp Kodon enthält und die Fragmente A und B so verbindet, daß sie ein nicht unterbrochenes offenes Leseraster bilden, welches in Fragment A beginnt und am Ende von Fragment B endet und so für ein Polypeptid kodiert.

Das Fragment C kann vorzugsweise bis zu 134 Nukleotide enthalten. Besonders bevorzugt enthält Fragment C 2 bis 68 Nukleotide. Ganz besonders bevorzugt wird als Fragment C die synthetische DNA deren Sequenz in SEQ ID No:9 wiedergegeben ist. Dieses Fragment verbindet in besonders vorteilhafter Weise das in Fragment A beginnende offene Leseraster mit dem des Fragments B.

Als Promotoren, welche dem Fragment A vorgeschaltet werden können, kommen alle Promotoren in Frage, die in Pflanzen wirksam sind. Unter diesen seien neben denen, die durch Polymerase II kontrolliert werden auch solche hervorgehoben, die unter der Kontrolle der Polymerase III stehen. Ebenso hervorgehoben werden Promotoren die aus pflanzenpathogenen Viren stammen. Besonders bevorzugt unter diesen wird der 35S-Promotor des Cauliflower Mosaikvirus (CaMV). Seine Sequenz ist in SEQ ID No:3 wiedergegeben. Er ist vorzugsweise direkt mit dem Fragment A verbunden.

Als 3'-untranslatierte DNA, die eine Polyadenylierungsstelle und ein TTS-Signal enthält und die dem Fragment B, gegebenenfalls unter Zwischenschaltung des Fragments D, folgt, können alle entsprechenden Regionen eingesetzt werden, die Polyadenylierung und Transkriptionstermination in Pflanzen bewirken. Vorzugsweise werden 3'-untranslatierte DNA-Sequenzen aus pflanzenpathogenen Viren gewählt. Besonders bevorzugt wird die 3'-untranslatierte CaMV 35S-DNA verwendet, deren Sequenz aus SEQ ID No:4 hervorgeht.

Die oben erläuterten Fragmente können in der erfindungsgemäßen rekombinanten DNA jeweils durch entsprechende Fragmente mit einer gleichwirkenden DNA ersetzt werden. In der gleichwirkenden DNA können 1 oder mehrere Basen oder 1 oder mehrere Kodonen fehlen oder durch andere Basen oder Kodone ersetzt vorliegen, wobei jedoch die Fragmente nur soweit verändert werden können, daß ihre Wirkung im wesentlichen erhalten bleibt, d.h. daß die erfindungsgemäßen Kombinationen der Fragmente in Pflanzen zu einer erhöhten Resistenz gegenüber Schadorganismen und Pflanzenkrankheiten führen. Als gleichwirkende DNA kann jede DNA betrachtet werden, die mit einer Einzelstrang DNA gemäß SEQ ID No:1-13 unter stringenten Bedingungen hybridisiert werden kann und in Kombination mit den übrigen Fragmenten in Pflanzen die gewünschte Resistenzerhöhung bewirkt. Gleichwirkende DNA ist vorzugsweise eine DNA, von der die gleichen oder "gleichwirkende" Polypeptide "abgeleitet" werden können, wie von den DNA-Sequenzen gemäß SEQ ID: 1, 5, 7, 9 und 11 (vgl. auch die Proteinsequenzen gemäß SEQ ID: 2, 6, 8, 10 und 12). Abgeleitet bedeutet hier, daß eine DNA in RNA transkribiert und diese in Protein translatiert werden kann. Gleichwirkende Polypeptide bedeuten, daß es sich um Polypetide handelt, deren gesamte Primärsequenz die gleiche Wirkung hat, wobei jedoch einzelne Aminosäuren ausgetauscht sein können ohne Sekundär-, Tertiär-und Quaternärstruktur in dem Sinne zu verändern, daß die gewünschte Resistenzerhöhung verloren geht. DNA gemäß der SEQ ID No:7 und 11 kann auch als Sonde benutzt werden um aus Scharkaviren oder Tospoviren entsprechende weitere Fragmente zu isolieren, die erfindungsgemäß verwendet werden können. Mit Hilfe dieser Sonden können auch in üblicher Weise die Fragmente in Vektoren oder im pflanzlichen Genom nachgewiesen werden.

Erfindungsgemäß bevorzugt ist die rekombinante DNA, welche wenigstens eines der Fragmente A oder B mit der Sequenz gemäß SEQ ID No:7 bzw. 11 oder eine entsprechend gleichwirkende DNA enthält.

Erfindungsgemäß besonders bevorzugt ist die rekombinante DNA, welche beide Fragmente A und B mit der Sequenz gemäß SEQ ID No:7 und 11 oder eine entsprechend gleichwirkende DNA enthält.

Erfindungsgemäß ganz besonders bevorzugt ist die rekombinante DNA mit der Sequenz gemäß SEQ ID No:1 einschließlich der entsprechenden gleichwirkenden DNA. Als vollständige Transkriptionseinheit ist die rekombinante DNA mit der Sequenz gemäß SEQ ID No:5 oder eine entsprechend gleichwirkende DNA ganz besonders bevorzugt.

Die erfindungsgemäß verwendbaren DNA Fragmente sowie die erfindungsgemäße DNA können nach den üblichen Methoden anhand der Information aus den SEQ ID No: 1-13 erhalten werden. Darüber hinaus sind erfindungsgemäß verwendbare cDNA Fragmente des Scharkavirus (PPV)-Genoms sowie cDNA Fragmente des S RNA Genoms des TSW Virus von der Deutschen Sammlung von Mikroorganismen und Zellkulturen (DSM) unter den Nummern PL-1080 (pPPV-NAT 5'-4) und PL-1081 (pTSW L3-308) erhältlich. Ebenso können verschiedene cDNAs des "Straßburg Isolates" und des Isolates CM1841 (ATCC No. 45031) von CaMV unter den Nummern PL-1029 und PL-1033 von der DSM erhalten werden (vgl. DSM-Bestelliste, Pflanzenvirus Gruppe, Messeweg 11/12, W-3300 Braunschweig, Bundesrepublik Deutschland).

Die Plasmide PL-1080 und PL-1081 wurden in Escherichia coli bei der Deutschen Sammlung von Mikroorganismen (DSM), Mascheroder Weg 1b, D-3300 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt:

| DSM-Bezugszeichen | Eingangsnummer | Hinterlegungsdatum |
|---|---|---|
| BY-1080 (enthält PL-1080) | DSM 8276 | 07.05.1993 |
| BY-1081 (enthält PL-1081) | DSM 8277 | 07.05.1993 |

Die erfindungsgemäße rekombinate DNA kommt in der Natur nicht vor. Sie kann anhand der in der vorliegenden Beschreibung enthaltenen Informationen, insbesondere unter zu Hilfenähme der SEQ ID No: 1 bis 13 durch den Fachmann mit Hilfe der üblichen Methoden der Molekularbiologie erhalten werden (Sambrook et al. (1989).

Die vorliegende Erfindung betrifft auch neue Vektoren insbesondere Plasmide oder Phagen, die die erfindungsgemäße rekombinante DNA enthalten, z.B. ( wie unten beschrieben) die Plasmide pSLTSWVL3 und pXLTSWVL3. Die vorliegende Erfindung betrifft auch neue Wirtsorganismen, insbesondere Mikroorganismen, die die erfindungsgemäße rekombinate DNA, vorzugsweise in ein Plasmid integriert, enthalten. Als Mikroorganismen kommen vor allem die üblicherweise verwendeten *Escherichia coli* Stämme und *Agrobacterium tumefaciens* Stämme in Frage. Beispielhaft sei der weiter unten beschriebene *Agrobacterium tumefaciens* Stamm XLTSWVL3 genannt, der das Plasmid pXLTSWVL3 enthält.

Die Vektoren und Mikroorganismenstämme können anhand der in der vorliegenden Beschreibung enthaltenen Informationen insbesondere unter zu Hilfenahme der Seq ID No: 1 bis 13 durch den Fachmann mit Hilfe der üblichen Methoden der Molekularbiologie erhalten werden.

Die erfindungsgemäße rekombinante DNA kann in das Genom von Pflanzen eingesetzt werden, wodurch eine erhöhte Resistenz dieser transgenen Pflanzen gegenüber Schadorganismen und Pflanzenkrankheiten erzielt wird.

Die erhöhte Resistenz der transformierten Pflanzen ist von Bedeutung für Landwirtschaft und Forsten, für den Zierpflanzenanbau, den Heilpflanzenanbau und die Pflanzenzucht. Auch bei der Kultivierung von Pflanzenzellen, z.B. zur Gewinnung von pharmazeutisch brauchbaren Stoffen, ist es von Vorteil, Pflanzenzellen verfügbar zu haben, welche gegen den Befall durch mikrobielle Schädlinge, insbesondere Pilze und Viren, erhöhte Resistenzen aufweisen.

Die vorliegende Erfindung betrifft somit auch ein Verfahren zur Herstellung transgener Pflanzenzellen (einschließlich Protoplasten) und Pflanzen (einschließlich Pflanzenteile und Samen) mit erhöhter Resistenz gegen Schadorganismen und Pflanzenkrankheiten, welches dadurch gekennzeichnet ist, daß man

4

(a) die erfindungsgemäße DNA in ein- oder mehrerfach in das Genom von Pflanzenzellen (einschließlich Protoplasten) einsetzt und gegebenenfalls

(b) aus den transformierten Pflanzenzellen (einschließlich Protoplasten) vollständige transformierte Pflanzen regeneriert und gegebenenfalls vermehrt und gegebenenfalls

(c) von den so erhaltenen transgenen Pflanzen der Elterngeneration oder weiterer daraus gewonnener Generationen die gewünschten Pflanzenteile (einschließlich Samen) gewinnt und,

d) gegebenenfalls, zur Erzeugung weiterer Pflanzen das Genom der transgenen Pflanzen mit dem Genom weiterer Pflanzen kombiniert und die Nachkommen vermehrt.

Die Verfahrensschritte (a), (b) und (c) können nach den bekannten Verfahren und Methoden in üblicher Weise durchgeführt werden.

Transgene Pflanzenzellen (einschließlich Protoplasten) und Pflanzen (einschließlich Pflanzenteile und Samen), welche ein oder mehrere Exemplare der erfindungsgemäßen DNA enthalten, sowie solche transformierte Pflanzenzellen und Pflanzen, welche nach den obigen Verfahren erhältlich sind, gehören ebenfalls zur vorliegenden Erfindung.

Teile der vorliegenden Erfindung sind auch die:

(a) Verwendung der erfindungsgemäßen rekombinanten DNA zur Erhöhung der Resistenz von Pflanzen gegenüber Schadorganismen und Pflanzenkrankheiten, die

(b) Verwendung der erfindungsgemäßen rekombinanten DNA zur Erzeugung von Pflanzenzellen, Pflanzen, Pflanzenteilen und Pflanzenvermehrungsmaterial mit erhöhter Resistenz gegenüber Schadorganismen und Pflanzenkrankheiten sowie

c) die Verwendung der erfindungsgemäßen transgenen Pflanzen (einschließlich Pflanzenteilen und Samen) zur Erzeugung von Vermehrungsmaterial sowie zur Erzeugung neuer Pflanzen und deren Vermehrungsmaterials.

Teil der vorliegenden Erfindung sind auch Pflanzen (einschließlich Pflanzenzellen, Pflanzenteilen und Vermehrungsmaterial), welche das Protein gemäß SEQ ID No: 2 bzw. 6 oder gegebenenfalls ein am Aminoterminus verkürztes Protein gemäß SEQ ID No: 2 bzw. 6 enthalten.

Eine Anzahl verschiedener Methoden steht zur Verfügung, die erfindungsgemäße rekombinante DNA in das genetische Material von Pflanzen bzw. Pflanzenzellen einzusetzen. Der DNA-Transfer kann nach den allgemein üblichen bekannten Methoden erfolgen, wobei der Fachmann die jeweils geeignete Methode ohne Schwierigkeiten ermitteln kann. Die erfindungsgemäße DNA, welche zur Transformation von Pflanzen verwendet werden soll, muß einen geeigneten in Pflanzen wirkenden Promotor und eine 3'-untranslatierte Region enthalten. Besonders geeignet ist die rekombinierte DNA gemäß SEQ ID No: 5.

Das Ti-Plasmid von Agrobacterium tumefaciens steht als besonders günstiger und breit einsetzbarer Vektor zur Übertragung von fremder DNA in Genome dikotyler und monokotyler Pflanzen zur Verfügung. Die erfindungsgemäße DNA wird zusammen mit regulatorischen DNA-Sequenzen in die T-DNA von geeigneten Ti-Plasmiden eingesetzt (z.B. Zambryski et al., 1983) und durch Infektion der Pflanze, Infektion von Pflanzenteilen oder Pflanzengeweben, wie z.B. von Blattscheiben, Stengeln, Hypokotylen, Kotyledonen, Meristemen und davon ableitenden Geweben, wie z.B. sekundären Embryonen und Kalli oder durch Kokultur von Protoplasten mit Agrobacterium tumefaciens übertragen.

Eine Alternative ist die Inkubation der DNA in Pflanzenprotoplasten (z.B. Hain et al., 1985; Krens et al., 1982; Paszkowski et al., 1984) in Gegenwart von Polykationen oder Calziumsalzen und Polyethylenglykol.

Die DNA-Aufnahme kann auch zusätzlich durch ein elektrisches Feld (Elektroporation) begünstigt werden (z.B. Fromm et al., 1986).

Die DNA kann in bekannter Weise auch über Pflanzenpollen eingeführt werden, indem Pollen mit physikalisch beschleunigten Partikeln "beschossen" werden, welche die DNA tragen (vgl. EP-A 0 270 356).

Die Regeneration der Pflanzen erfolgt in bekannter Weise mit Hilfe geeigneter Nährmedien (z.B. Nagy und Maliga 1976).

In einer bevorzugten Ausführungsform des erfindungsgemäßen Verfahrens (gemäß der Methode aus EP-A 116 718) wird die erfindungsgemäße rekombinante DNA in isolierter Form in einen geeigneten intermediaeren E.coli Vektor z.B. pGV700 oder pGV710, (vergl. EP-A-116 718) bzw. vorzugsweise Derivaten davon, die zusätzlich ein Reportergen wie z.B. nptII (Herrera-Estrella et al. 1983) oder hpt (Van den Elzen et al 1986) enthalten, kloniert.

Das so konstruierte Plasmid wird auf Agrobacterium tumefaciens, das z.B. pGV 3850 bzw. Derivate davon enthält (Zambryski et al. 1983) mit üblichen Methoden (z.B. Van Haute et al. 1983) übertragen. Alternativ dazu kann die erfindungemäße DNA in einem binaeren Vektor, z.B. pCV001 oder pCV002 (z.B. Koncz und Schell 1986) kloniert und wie oben beschrieben in einen geeigneten Agrobakterium Stamm (Koncz und Schell 1986) transferiert werden. Der resultierende Agrobakterium Stamm, der die rekombinante DNA in einer auf Pflanzen transferierbaren Form enthält wird im weiteren zur Pflanzentransformation verwendet.

In einer weiteren bevorzugten Ausführungsform wird die rekombinante DNA gegebenenfalls zusammen mit einem anderen Plasmid, das ein Reportergen für Pflanzenzellen, z.B. für Kanamycin-Resistenz (z.B. Herrera-Estrella et al. 1983) oder eine Hygromycin-Resistenz (van den Elzen, 1986) enthält, vorzugsweise pLGV neo 2103 (Hain et al. 1985), pMON 129 (Fraley R.T. et al., Proc. National Acad. Sci. USA $\underline{80}$, 4803 (1983)), pAK 1003, pAK 2004 (Velten J. et al., EMBO Journ. Vol. 3, 2723 (1984)) oder pGSST neo 3 (pGSST3) (EP-A-189 707), in üblicher Weise durch direkten Gentransfer auf Pflanzenprotoplasten übertragen (z.B. Hain et al 1985). Dabei können das bzw. die Plasmide in zirkulärer, vorzugsweise jedoch in linearer Form, vorliegen. Bei der Verwendung eines Plasmids mit Reportergen werden kanamycinresistente Protoplasten dann auf Expression der rekombinanten DNA überprüft. Im anderen Fall (ohne Reportergen) werden die resultierenden Kalli auf die Expression der rekombinanten DNA geprüft (Screening mit üblichen Methoden).

Transgene Pflanzen bzw. Pflanzenzellen werden nach den bekannten Methoden, z.B. durch Blattscheiben Transformation (z.B. Horsch et al. 1985) durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycin-sulfat in vitro (Reiss et al. 1984; Schreier et al. 1985) oder durch die Expression der Nopalinsynthase (nach Aerts et al. 1983) oder der erfindungsgemäßen rekombinanten DNA durch Northern-Blot-Analyse und Western Blot-Analyse nachgewiesen. Die Genprodukte der rekombinanten DNA können auch in bekannter Weise mit Hilfe spezifischer Antikörper in transformierten Pflanzen nachgewiesen werden (Adam et al. 1987, 1991).

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transformierten Pflanzenzellen als auch die transformierten Pflanzen, welche die erfindungsgemäße DNA enthalten und welche Bestandteile der vorliegenden Erfindung sind, zeigen eine erheblich höhere Resistenz gegen Schadorganismen und Pflanzenkrankheiten, insbesondere gegen Nematroden pflanzenpathogene Pilze und Viren.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w. "Pflanzenzellen" schließen Protoplasten, Zelllinien, Pflanzenkalli usw. ein. "Vermehrungsmaterial" bedeutet Pflanzen, Pflanzenteile, wie Samen, Knollen und Stecklinge sowie Pflanzenzellen, welche zur Vermehrung der transgenen Pflanzen und Pflanzenzellen verwendet werden können und ist somit ebenfalls Teil der vorliegenden Erfindung.

Zu den Pflanzen, welchen durch den Einbau (Transformation) der erfindungsgemäßen rekombinanten DNA eine erhöhte Resistenz gegenüber den Schädlingen verliehen werden kann, gehören praktisch alle Pflanzen. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernden Pflanzen, z.B. Getreide (insbsondere Weizen, Roggen, Gerste, Hafer, Hirse, Reis und Mais), Kartoffel, Leguminosen (wie Hülsenfrüchte und insbesondere Alfalfa, Sojabohnen), Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen wie Rauwolfia und Digitalis. Besonders bevorzugt seien Kartoffel, Tomaten, Leguminosen und Curcubitaceen genannt.

Als Schädlinge (Schadorganismen und Pflanzenkrankheiten hervorrufende Organismen), gegen welche mit Hilfe der erfindungsgemäßen DNA erhöhte Resistenzen erzielt werden können, seien tierische Schädlinge, wie Insekten, Milben und Nematoden sowie mikrobielle Schädlinge, wie phytopathogene Pilze, Bakterien und Viren genannt. Besonders hervorgehoben werden pflanzenschädigende Nematoden sowie mikrobielle Schädlinge, insbesondere phytopathogene Pilze und Viren.

Zu den schädlichen Insekten gehören insbesondere Insekten der Ordnungen: Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere: Tarsonemus spp., Panonychus spp. und Tetranychus spp.

Zu den pflanzenparasitären Nematoden gehören Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp.,

Zu den mikrobiellen Schädlingen geboren insbesondere die phytopathogenen Pilze: Plasmodiophoromycetes, Oomycetes, Chytridiomycetes, Zygomycetes, Ascomycetes, Basidiomycetes,

EP 0 626 449 A2

Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Zu den Viruserkankungen gehören insbesondere Mosaik-, Verzwergungs- und Vergilbungsvirosen und Virosen, die durch Tospoviren hervorgerufen werden.

Beispielhaft aber nicht begrenzend seien einige Erreger von virösen, pilzlichen und bakteriellen Erkrankungen, die unter die oben aufgezählten Oberbegriffe fallen, genannt:

Barley Yellow Dwarf Virus (BYDV), Potato Virus Y (PVY), Cucumber Mosaic Virus (CMV), Watermelon Mosaic Virus (WMV), Tristeza-Virus, Tobacco Mosaic Virus (TMV), Tobacco Necrosis Virus (TNV), Beet necrotic Yellow Vein Virus (BNYVV) und insbesondere Tomato spotted wilt virus (TSWV), alle Tospoviren, Tabacco Mosaic virus (TMV) und andere Tobamoviren, Tabaccorattle virus und andere Tobraviren.

Xanthomonas-Arten, wie beispielsweise Xanthomonas campestris pv. oryzae;

Pseudomonas-Arten, wie beispielsweise Pseudomonas syringae pv. lachrymans;

Erwinia-Arten, wie beispielsweise Erwinia amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielwseise Pseudocercosporella herpotrichoides.

Weiterhin sei Helminthosporium carbonum aufgeführt.

Literatur:

Adam G, Chagas CM, Lesemann DE; (1987) *J of Phytopathology* **120**, 31-43

Adam G, Lesemann DE, Vetten HJ (1991) *Ann appl Biol* **118**, 87-114

Aerts M. et al., Plant Sci. Lett. 17 (1993), 43-50

Beachy RN, Chen Z-L, Horsch RB, Rogers SG, Hoffmann NJ, Fraley RT (1985) *EMBO J* **4**, 3047-3050

de Avila AC, de Haan P, Kormelink R, Resede RdeO, Goldbach RW, Peters D (1993) *J of Gen Virol* **74**, 153-159

Chomezynski et al., Anal. Bioch. 162 (1987), 156-159

de Haan P, Wagemakers L, Peters D, Goldbach R (1990) *J Gen Virol* **71**, 1001-1007

de Haan P, Gielen JJL, Prins M, Wijkamp IG, van Schepen A, Peters D, van Grinsven MQJM, Goldbach R (1992) *Bio/technologie* **10**, 1133-1137

Fromm ME et al., Nature 319 (1986), 791-793

Gielen JJL, de Haan P, Kool AJ, Peters D, van Grinsven MQJM, Goldbach R (1991) *Bio/technologie* **9**, 1363-1367

Goodall et al., Meth. in Enzym. 181 (1990), 148-161

Goodall and Filipowicz, Cell 58 (1989), 473-483

Herrea-Estrella et al., Nature 303 (1983), 209-213

Hain R et al., Molec. Gen. Genet. (1985) 199, 161-168

Herrera-Estrella L et al., EMBO J2 (1983), 987-995

Hoekema A, Hirsch PR, Hooykaas PJJ, Schilperoort RA (1983) *Nature* **303**, 179-180

Horsch RB et al., Science 277 (1985), 1229-1231

Krens FH et al., Nature 296 81982), 72-74

Koncz C, Schell J, Mol. Gen. Genet. 204 (1986), 338-396

Landsmann J, Llewwellyn D, Dennis ES, Peacock WJ (1988) *Mol Gen Genet* **214**, 68-73

MacKenzie DJ, Ellis PJ (1992) *Molecular Plant—Microbe Interactions* **5**, 34-40

Maiss E, Timpe U, Brisske A, Jelkmann W, Casper R, Himmler G, Mattanovich D, Katinger HWD (1989) *J of Gen Virol* **70**, 513-524

Maiss E, Ivanova L, Breyel E, Adam G (1991) *J of Gen Virol* **72**, 461-464

Maiss E, Timpe U, Brisske-Rode A, Lesemann D-E, Casper R (1992) *J of Gen Virol* **73**, 709-713

Marton L et al., Nature (1979), 1229-1231

Mullis KB, Faloona FA (1987) *Methods Enzymol* **155**, 335-350

Murashige, T., Skoog F., Physiol. Plant. 15 (1962), 47

Paszkowski J. et al., EMBO J3 (1984), 2717-2722

Pietrzak et al. (1986) *Nucleic Acid Res.* **14**, 5857-5868

Powell Abel P, et al. (1986) *Science* **232**, 738-743

Reiss B et al., GENE 1081 (1984), 211-217

Sambrook J, Fritsch EF, Maniatis T (1989) *Molecular Cloning a Laboratory Manual* 2[d] Edition

Schreier P et al., EMBO J4 (1985), 25-32

Sheng-Zi et al. Phytopathology 82 (1992), 1223-1229

Steinecke et al., EMBO J11 (1992), 1525-1530

Töpfer R, Matzeit V, Gronenborn B, Schell J, Steinbiss HH (1987) *Nucl Acid Res* **15**, 5890

Tumer NE, O'Connell KM, Nelson RS, Sanders PR, Beachy RN, Fraley RT, Shah DM (1987) *EMBO J* **6**, 1181-1188

Van den Elzen PJM et al., Plant Mol. Biol. 5 (1985), 299-302

Van Haute E et al., EMBO J2 (1983), 411-418

Velten J et al., EMBO J12 (1984), 2723-2730

Zambryski et al., EMBO J. 2 (1983), 2143-2150.

Die vorliegende Erfindung soll anhand der folgenden Beispiele erläutert werden:

1. Konstruktion der rekombinanten DNA und Übertragung in Agrobakterien:

Wie bereits ausgeführt, betrifft die vorliegende Erfindung eine neue rekombinate DNA, insbesondere ein rekombinantes chimäres Gen (SEQ ID No: 1), welches unter der Kontrolle eines Promotors steht und eine 3'-ständige Sequenz enthält. Als Beispiel werden hier der CaMV35S Promotor (5'-Region) (SEQ ID No: 3) und die 3'- untranslatierte Region (SEQ ID No: 4) des CaMV 35S-Transkripts verwendet (Töpfer et al.1987 und EP-A2-0 223 452). Das gesamte hier verwendete Beispiel ist in SEQ ID No: 5 gezeigt. Die erfindungsgemäße DNA (chimäres Gen) (SEQ ID No: 1) besteht aus mehreren Stücken, die in bestimmter Reihenfolge zusammengefügt sind. Es besteht aus einem doppelsträngigen cDNA Fragment von 152 Basenpaaren welche von der RNA des Scharkavirus (Plumpox Virus (PPV))(Maiss et al., 1989) abgeleitet wurde. Dieses entspricht der viralen RNA Sequenz der Positionen 1-152 (SEQ ID No: 7) (Maiss et al., 1989). In diesem Fragment beginnt ein offenes Leseraster welches über ein synthetisches cDNA Fragment (SEQ ID No: 9) mit einem cDNA Fragment der S-RNA des L3 Isolates des TSWV N-Gens (Maiss et al.; 1991)(SEQ ID No: 11) verbunden ist. Diese Verbindung ist dergestalt, daß ein offenes Leseraster von insgesamt 956 Basen Länge ohne Unterbrechungen entsteht, welches bei der Position 992 der "SEQ ID No: 1" endet. Die darauf folgende cDNA Sequenz der S RNA des 3'- nicht translatierten Bereichs des TSWV (SEQ ID No: 11) ist über eine synthetische Sequenz (SEQ ID No: 13) mit der 3'-Region des 35S Transkriptes des CaMV (SEQ ID No: 4) (Töpfer et al.; 1987) verbunden.

Die Konstruktion erfolgte mit Methoden die allgemein bekannt sind und deren Details in (Sambrook,Fritsch und Maniatis, 1989) beschrieben sind.

Im Einzelnen wurde wie folgt verfahren. Ein HindIII/BamHI Fragment von 454 Basenpaaren Länge aus dem käuflichen Plasmid pCaMVCN (Pharmacia) wurde in das käufliche Plasmid pT7T3 19U (Pharmacia) kloniert und pT7T3 35S genannt. Dieses neue Plasmid wurde mit SalI geschnitten, die überstehenden Enden aufgefüllt und die DNA danach mit EcoRI geschnitten. In dieses, so vorbereitete Plasmid, wurde ein DraI/EcoRI Fragment (572 Basenpaare Länge )aus dem Plasmid pPPV-NAT 5'4 (Maiss et al., 1989; Maiss et al. 1992) einkloniert. Dieses Fragment enthält eine cDNA Kopie des 5'-Endes des PPV. Das neue Plasmid wurde p35S 5'.16 genannt.

Mit dem synthetischen Oligonukleotid 5'-TGTGTTGAGTTTTTATATTTTCCTCTCCAAATGAAA-3' (SEQ ID No: 14), welches komplementär zum nicht kodierenden Strang ist, wurde mit Hilfe der *in vivo* Mutagenese das 3'-Ende der CaMV35S Promotorsequenz (5'-...AGAGG-3')(SEQ ID No: 15) an das äußerste 5'-Ende (5'-AAAATAT....-3')(SEQ ID No: 16) der cDNA der PPV - Sequenz gebracht . Das resultierende neue Plasmid wurde p35S genannt.

Aus diesem Plasmid wurde ein HincII Fragment von 568 Basenpaare Länge isoliert und in das käufliche Plasmid pT7T3 19U, welches mit SmaI und HincII vorgeschnitten war, einkloniert. Das resultierende Plasmid heißt pT7T3 19UPPVL. Dieses Plasmid wurde mit EcoRV und SalI geschnitten und ein Fragment von 239 Basenpaare Länge isoliert. Dieses Fragment wurde in die EcoRV, XhoI Schnittstellen von pRT101 (Töpfer et al. 1987) kloniert. Das resultierende Plasmid wurde pSL genannt.

Aus dem Plasmid pTSWV-L3/308 (Maiss et al.; 1991) wurde ein BamHI/HindIII Fragment isoliert. Die Enden wurden aufgefüllt und in die SmaI Schnittstelle des Vektors pRT101 (Töpfer et al. 1987) eingesetzt. Das Plasmid, welches das eingefügte Fragment in der richtigen Orientierung enthält wurde pSTSWVL3 genannt. Aus diesem Plasmid wurde ein RsaI/BamHI Fragment von 868 Basenpaaren Länge isoliert und in den oben erwähnten Vektor pSL, der mit SmaI und BamHI geschnitten war, einkloniert. Das resultierende neue Plasmid wurde pSLTSWVL3 genannt. Aus diesem kann mit Hilfe von HindIII ein Fragment von 1716 Basenpaaren Länge isoliert werden, welches das gesamte chimäre Gen und die 5'- und 3'-untranslatierten Regionen des CaMV (35S) enthält. Dieses Fragment wurde in das Plasmid pXL222 (Landsmann et al. 1988), welches mit HindIII geschnitten war einkloniert. Das resultierende Plasmid wurde pXLTSWVL3 genannt. Dieses Plasmid (pXLTSWVL3) wurde in den *A. tumefaciens Stamm* LBA 4404 (Hoekema et al. 1983) mobilisiert oder transfiziert. Dieser neue Agrobakterienstamm wurde XLTSWVL3 genannt.

2. Erzeugung von transgenen Pflanzen und Nachweis der Expression des chimären Gens in diesen Pflanzen

Der Agrobakterienstamm XLTSWVL3 wurde zur Transformation von Pflanzenzellen oder Pflanzenteilen benutzt.

Die transgenen Pflanzen wurden mit Hilfe eines gleichzeitig übertragenen Markierungs- und Selektionsgenes, dem Gen für Kanamycinresistenz, auf kanamycinhaltigem Medium selektioniert. Einundzwanzig individuelle Kandidaten wurden nach ihrer Fähigkeit das chimäre Gene zu exprimieren ausgewählt. Dabei wurde zuerst die Genexpression auf der Ebene der mRNA bestimmt. Hierzu wurde die aus transgenen Pflanzen isolierte, gesamte RNA mit Hilfe der reversen Transkriptase und dem synthetischen Oligonukleotid 5'-GTC AGT GGC TCC AAT CCT GTC TGA AG - 3' (SEQ ID No: 17) in cDNA übersetzt und das Vorhandensein von mRNA des chimären Gens mit Hilfe eines zweiten synthetischen Oligonukleotids 5' - TGT CGA GAA TTC GAG CTC GGT AC - 3' (SEQ ID No: 18) und der Polymerase Kettenreaktion (PCR; Mullis und Faloona, 1987) vermehrt und als Fragment von 328 Basenpaaren Länge identifiziert.

Die einundzwanzig transgenen Pflanzen, bei denen in der oben beschriebenen Art und Weise die Genexpression nachgewiesen wurde, wurden nun mit Hilfe eines spezifischen Antikörpers (ELISA) (Adam et al.,1987; 1991) auf die Produktion des chimären Genprodukts hin überprüft. Die Kandidaten XL4, XL8, XL11, XL14, XL15 und XL19 zeigten ein deutlich messbares Signal und wurden für erste orientierende phytopathologische Experimente ausgewählt.

Die Transformation von Pflanzen wurde im Detail, wie folgt durchgeführt.

2.1 Agrobakterium-Transformation

Für die Transformation von Tabak wurden Blattscheiben (Horsch et al., 1985) verwendet. Dazu wurden ca. 2-3 cm lange Blätter von sterilen Sproßkulturen in Scheiben von 1 cm Durchmesser gestanzt und mit einer für 24 Stunden gezüchteten Agrobakterium-Kultur nach Aufnahme in 10 mM MgSO$_4$ und in 20 ml MS-Medium (Murashige und Skoog, 1965) für etwa 60 Stunden bei 26°C inkubiert. Die Blattscheiben wurden anschließend 3x in MS-Medium mit 1000 $\mu$g/ml Claforan gewaschen und auf MS-Platten, die 0,5 $\mu$g/ml NAA (Naphthylessigsäure), 0,2 $\mu$g/ml BAP (Benzylaminopurin) und 2 % Saccharose ("Kallusinduktionsmedium") und das entsprechende selektionierende Agens (z.B. Kanamycinsulfat) enthielten, ausgelegt und bei 26°C inkubiert. Nach jeweils 2 Wochen wurden die Blattscheiben auf frisches Kallusinduktionsmedium überführt. Nachdem kleine Kalli zu erkennen waren, wurden diese auf Sproßinduktionsmedium überführt, das dem Kallusinduktionsmedium entspricht, jedoch 0,2 $\mu$g/ml NAA und 0,5 $\mu$g/ml BAP enthält. Nachdem die regenerierten Sprosse eine Länge von 2-3 cm aufwiesen, wurden sie zur Wurzelinduktion auf MS-Medium, das 1 % Saccharose aber keine Hormone enthielt, überführt und unter sterilen Bedingungen bei 24 - 26°C (12 Std. Licht (1000 - 3000 Lux), 12 Std. Dunkel) kultiviert. Nach 4 bis 6 Wochen wurden die Sproßkulturen auf frisches Medium überführt, zu Pflanzen herangezogen und anschließend unter geeigneten Bedingungen im Gewächshaus kultiviert.

Für die Transformation von Kartoffelpflanzen erfolgte nach der Inkubation der ausgeschnittenen Blattscheiben in der Agrobakterium-Suspension eine weitere Inkubation für zwei Tage in Dunkelheit. Die Blattscheiben wurden anschließend auf MS-Platten, die 1,6% Glucose, 5mg/l NAA, 0,1mg/l BAP, 500 mg/l Claforan und das entsprechende selektionierende Agens enthielten, für 8 Tage bei 16 stündiger Belichtung inkubiert. Anschließend wurden die Blattscheiben auf MS-Platten überführt, die 1,6% Glucose, 2,0 mg/l Zeatinriboside, 0,02 mg/l NAA, 0,02 mg/l GA3 (Giberellinsäure) und 500 mg/l Claforan enthielten. Nach 14 tägiger Inkubation wurden die Blattscheiben auf frische Platten überführt. Erste Kalli erschienen nach etwa 6 bis 7 Wochen. Erscheinende Sprosse wurden in 250 ml Glaskolben, die MS-Medium mit 3% Saccharose und 0,5 mg/ml Carbenicillin enthielten, überführt. Erste Wurzeln erschienen nach etwa 14 Tagen. Die Pflanzen wurden anschließend unter geeigneten Bedingungen im Gewächshaus kultiviert.

Die Transformation von Tomaten erfolgte im wesentlichen nach dem vorher beschriebenen Verfahren.

Aus den erhaltenen Pflanzen wurde nach Standardverfahren DNA (Dellaporta et al., 1983) und RNA (Goodall et al., 1990, Chomczynski und Sacchi, 1987) isoliert. Mit Hilfe der DNA-Analyse ("Southern-Blot") konnte die Integrität und die Anzahl der übertragenen Gene, und mit Hilfe der RNA-Analyse ("RNase-Protection" nach Vankan und Filipowicz, 1988; Goodall und Filipowicz, 1989; Steinecke et al., 1992) konnte die Transkriptmenge bestimmt werden.

2.2 Protoplasten-Isolation und PEG vermittelte Transformation

4-8 Wochen alte *Nicotiana tobacum* SR1 Blätter aus einer Sterilkultur wurden abgeschnitten, mit $H_2O$ dest. gereinigt und in eine 0,05 % SDS-Lösung gelegt, um Bakterienkontaminationen zu vermeiden. Das SDS wurde durch zweimaliges Waschen mit sterilem $H_2O$ entfernt und die Blätter 10 min in K3-Medium (500 mg/l Claforan, 1 mg/l NAA, 0,2 mg/l Kinetin) äquilibriert. Nach Entfernung der Mittelrippe wurden die Blätter in 1-2 $cm^2$ große Stücke geschnitten. Zur Isolation der Mesophyll-Protoplasten wurden die Blattstücke in eine sterile 1 l Flasche mit 30 ml einer Cellulase und Mazerozym enthaltenden Enzymlösung (1,5 % Cellulase, 0,5 % Mazerozym, in K3-Medium) gegeben und für 10 min einem leichten Vakuum ausgesetzt. Nach 16 h Inkubation bei 27°C im Dunkeln, wurde der Ansatz 30 min bei 75 Upm geschüttelt. Die Protoplastensuspension wurde dann über Stahlsiebe mit 250 und 100 $\mu$m Porenweite gegeben, das Filtrat in 12 ml Zentrifugenröhrchen verteilt und 5 min bei 650 Upm in einer Hettich-Zentrifuge Universal 2S zentrifugiert. Mit einer 100 $\mu$m Glaskapillare wurde die untere Phase mit dem Niederschlag abgesogen, die Protoplasten mit 10 ml K3-Medium gewaschen und das Medium nach erneuter Zentrifugation (s.o.) wieder abgesaugt. Anschließend wurden die Protoplasten in W5-Medium (154 mM NaCl, 125 mM $CaCl_2xH_2O$, 5 mM Glucose, 5 mM KCl, pH 5,6) aufgenommen, in einer Fuchs-Rosenthal-Zählkammer gezählt und 30 min in W5-Medium stehen gelassen. Die Protoplasten wurden durch 5 min Zentrifugation bei 650 Upm abgetrennt und der Niederschlag in MaMg-Lösung (450 mM Mannitol, 15 mM $MgCl_2$, 0,1 % MES, pH 5,6) suspendiert, wobei eine Dichte von $1-3x10^6$ Protoplasten/ml eingestellt wurde. Die Protoplasten konnten sofort transfiziert oder bis zu 4 h im Kühlschrank aufbewahrt werden.

Für die Transformation wurden die Protoplasten einem 5 minütigen Hitzeschock unter gelegentlichem Schwenken bei 45°C im Wasserbad unterzogen und anschließend sofort 45 s in Eis/Wasser auf Raumtemperatur abgekühlt. Anschließend wurden je 0,35 ml Protoplastensuspension auf 10 ml Zentrifugenröhrchen verteilt und die DNA-Lösung in einem Volumen von 50 $\mu$l hinzugegeben. Nach 10 min wurden 0,35 ml PEG-Lösung (100 mM $Ca(NO_3)_2x4H_2O$, 400 mM Mannitol, 40 % PEG 4000, pH 7-9) hinzugetropft, und nach weiteren 20 min, in denen die Ansätze alle 5 min geschwenkt wurden, erfolgte die Überführung des Transfektionsansatzes in Petrischalen. Nach der tropfenweise Zugabe von 4 ml K3-Medium wurden die Protoplasten 6-48 h bei 27°C im Dunkeln kultiviert. Im Anschluß an die Inkubation wurden die Protoplasten in 12 ml Zentrifugenröhrchen gegeben, die zum Waschen mit Seewasser gefüllt waren, und 3 min bei 650 Upm zentrifugiert. Der Überstand wurde bis auf 1 ml abgenommen und die Protoplasten nach Resuspension in Eppendorfgefaße überführt. Nach 1 min Zentrifugation bei 13000 Upm (Biofuge) und vorsichtigem Abnehmen des Überstandes wurden die Protoplasten in die jeweiligen Extraktionspuffer aufgenommen.

In nach der vorstehend beschriebenen Methode hergestellten Protoplasten konnten die erfindungsgemäße DNA eingebracht werden.

3. Beispiele für die erhöhte Resistenz erfindungsgemäßer transgener Pflanzen

3.1 Erhöhte Resistenz gegenüber pflanzenpathogenen Pilzen Die unter 2 (oben) ausgeführten transgenen Tabakpflanzen XL4, XL8, XL11, XL14, XL15 und XL19 wurden geselbstet und Nachkommen von XL4 für den folgenden Test ausgewählt. Als Testpathogen diente der pflanzenpathogene Pilz *Botrytis cincera.*

(a) Testbeschreibung

Die Tabakpßanzen wurden bei 23°C und 70-80% rel. Luftfeuchte im Gewächshaus bis Versuchsbeginn angezogen. Die Versorgung mit Wasser und Dünger erfolgt nach Bedarf. Zur Inokulation wurden die Blätter der 6-8 Wochen alten Pflanzen mit Sporensuspension des Pathogens tropfnass angesprüht. Anschließend wurden die Pflanzen unter für das Pathogen günstigen Bedingungen bei 100% rel. Luftfeuchte und 20°C inkubiert. Nach 4 Tagen wurde der Gesundheitszustand der Pflanzen anhand der befallenen Blattfläche in Prozent ermittelt. Es wurde nach Blattetagen differenziert (Blatt 1 = ältestes Blatt, Blatt 6 = jüngstes Blatt).

(b) Testergebnisse

| Pflanzen (je 47 Stück) | 1 - 3 Blatt | 4 - 6 Blatt | Mittelwert |
|---|---|---|---|
| *N.tabacum* (Wiltyp); % befallenen Blattflächen | 22,4 | 24,0 | 23,2 |
| Transgene Pflanzen; % befallenen Blattfläche | 14,3 | 17,1 | 15,7 |
| Befallsreduktion in % (Nach Abbott) | 36,3 | 29,0 | 32,5 |

3.2 Erhöhte Resistenz gegenüber Viren

Die unter 2 (oben) ausgeführten transgenen Tabakpflanzen XL4, XL8, XL11, XL14, XL15 und XL19 wurden geselbstet und die homozygoten Nachkommen von XL4 (XL4.24) und XL8 (XL8.28), für den folgenden Test ausgewählt.

Als Testpathogen dienten verschiedene Tospoviren und Viren anderer Familien.

(a) Testbeschreibung

Die Tabakpflanzen wurden bei 23°C und 70-80% rel. Luftfeuchte im Gewächshaus bis Versuchsbeginn angezogen. Die Versorgung mit Wasser und Dünger erfolgt nach Bedarf.

Von Tabakpflanzen (*Nicotiana rastica* L.), die 3 Wochen zuvor mit dem TSWV-Isolat L3 (DSM-Nr.: PV0182) infiziert wurden, wurden 3 g Blattmaterial von primär infizierten Blättern mit deutlichen Virussymptomen geerntet und in 30 ml Standard-Puffer (0,1 M Na-K-Phosphatpuffer pH 7,0 mit 0,2 % Polyvinylpyrrolidon MW 10000 und 0,2 % (w/v) $Na_2SO_3$) im Mörser homogenisiert. Dieses Material wurde unverdünnt und in einer 1:25 Verdünnung im Standard-Puffer verwendet und jeweils 0,1 ml pro Blatt auf je 3 Blätter einer etwa 8-10 Wochen alten Testpflanze mechanisch durch Abreiben appliziert. Die Testpflanzen wurden vorher mit einem Abrasiv (Karborundum 600 mesh) bestäubt und nach erfolgter Inokulation mit Leitungswasser abgespült.

Die Symptomentwicklung auf den Pflanzen wurde ab dem 5. Tag nach Inokulation über einen Zeitraum von 14 Tagen mit einem Boniturschema visuell beurteilt. Symptome treten auf den infizierten Blättern sowie systemisch auf nicht infizierten Blättern auf. Stufe 0 = symptomlos
Stufe 1 = Schwache Symptome, Ringflecken auf den Blättern (Nekrosen)
Stufe 2 = Starke Symptome, Adernvergilbung, Mosaik, Absterbeerscheinungen
Aus den Boniturwerten wurde der mittlere Boniturwert ermittelt und in Relation zu den Boniturwerten der Kontrollpflanzen gesetzt.

Zur weiteren Quantifizierung der Widerstandsfähigkeit der Pflanzen wurde ein Triple-Antibody-Sandwich (TAS)-ELISA-Verfahren zur Bestimmung der TSWV Antigenmenge verwendet. Dazu wurden Greiner Microtiterplatten (Typ 655001) pro Vertiefung mit 0,1 ml polyklonalem Antiserum (DSM-Nr., AS-0105, 2 μg/ml) beschichtet. Preßsäfte der Testpflanzen wurden von primär- und sekundärinfizierten Blättern in Verdünnungen 1:30 und 1:500 mit dem Standard-ELISA-Puffer hergestellt. Je 3 Vertiefungen der Platte wurden mit jeder Probe (0,1 ml) beschickt und über Nacht bei 4°C inkubiert. Nach dreimaligem Waschen mit PBS-Tween wurden je Vertiefung 0,1 ml einer 1:1000 Verdünnung der beiden monoklonalen Antikörper 4F2 und 2B6 (Adam et al., 1991) zugegeben und 3 h bei 37°C inkubiert. Nach dreimaligem Waschen mit PBS-Tween wurden pro Vertiefung 0,1 ml einer 1:1000 Verdünnung eines Kaninchen-Anti-Maus IgG-Konjugates (DAK Diagnostica GmbH, Nr.: D314) zugegeben und 3 h bei 37°C inkubiert. Nach dreimaligem Waschen mit PBS-Tween wurde das Substrat p-Nitrophenylphosphat (1 mg/ml) zugegeben und nach 30 min sowie 1 h die Extinktion bei 405 nm im Photometer gemessen. Aus den Extinktionswerten wurde der mittlere Extinktionswert ermittelt und in Relation zu den Kontrollmeßwerten gesetzt. Die Ergebnisse zeigen, daß die transgenen Pflanzen symptomlos sind, während die Kontrollpflanzen starke Symptome zeigen. Darüberhinaus zeigt sich, daR die TSWV-Antigenmenge in den transgenen Pflanzen, nachgewiesen durch die Reaktion mit dem monolonalen Antikörper (MAB 2B6), deutlich geringer ist als in den Kontrollpflanzen.
(b) Testergebnis

| Pflanzen (je 4X10) | Symptomstärke | Bemerkung |
|---|---|---|
| *N.tabacum* (Wiltyp) | 2 | Die Kontrollpflanzen sind nur zur halben Höhe der transgenen Pflanzen aufge- wachsen. |
| Transgene XL 4.24 | 0 | |
| Transgene XL 8.28 | 0 | |

Biotest von XL 4.24 transgenen Pflanzen mit verschiedenen anderen Tospovirusisolaten
(a) Testbeschreibung
Homozygote Nachkommen der transgenen Pflanze XL4 (XL 4.24) wurden so lange herangezogen, bis sie das Vierblattstadium erreicht hatten.

Die Pflanzen wurden in Gruppen von 20 zufällig zusammengefaßt und mechanisch mit Homogenaten aus mit verschiedenen Tospoviren infizierten Tabakpflanzen inokuliert. Folgende Virusisolate wurden für die Inokulation verwendet: TSWV-peanut = SA05 (DSM No. PV-0205); INSV (DSM No. PV-0280); INSV (DSM No. PV-0281). Die Pflanzen zur Gewinnung des Inokulums waren 10-14 Tage vorinokuliert und nach elektronenmikroskopischen Befund frei von Kontaminationen mit anderen Viren. Für die Herstellung des Inokulums wurde der Standard DSM Inokulationspuffer verwendet (DSM-Katalog Pflanzenviren), die Homogenisation erfolgte mit Hilfe von Mörsern und Pistill im Verhältnis 1:30 (g/ml). Das Inokulum wurde mit sterilen Glasspateln auf die mit Carborundum (600 mesh) bestäubten Blätter der Testpflanzen abgerieben. Von jeder Testpflanze wurden die zwei ältesten Folgeblätter inokuliert. Nach der Inokulation wurden die infizierten Blätter mit Leitungswasser abgewaschen. Anschließend wurden die Pflanzen unter den oben angeführten Kulturbedingungen inkubiert und täglich auf Symptome visuell boniert. Als Kontrollen wurden nicht transformierte *N. tabacum* Pflanzen in gleicher Weise angezogen und mit den verschiedenen Isolaten inokuliert.

Die endgültigen Bonituren des Versuches erfolgten eine und drei Wochen nach der Inokulation durch zwei Personen, bei ungleicher Bewertung wurde die abwertendere Bewertung herangezogen. Die Ergebnisse sind in Tabelle 1 zusammengefaßt. Das Isolat TSWV-peanut (SA-05) führte bei erfolgreicher Infektion zu lokalen Nekrosen auf den infizierten Blättern und zu starken systemischen Symptomen mit Blattdeformationen und Nekrosen auf den neugebildeten, systemisch infizierten Blättern, während die beiden INSV Isolate nur auf den inokulierten Blättern zur Ausbildung von lokalen Nekrosen führten während eine systemische Ausbreitung auf neugebildete Blätter unterblieb. Bei der Bonitur wurden im Falle von TSWV-peanut sowohl systemisch infizierte, als auch solche Testpflanzen als erfolgreich infiziert betrachtet, die nur lokale Primärsymptome zeigten, da erfahrungsgemäß hier mit zeitlicher Verzögerung auch systemische Symptome auftreten. Bei den beiden INSV Isolaten wurde nur das Auftreten von lokalen Primärsymptomen als erfolgreiche Infektion bewertet.

Tabelle 1

| Test der transgenen Tabaklinie XL4 (XL4.24) auf Resistenz gegenüber TSWV-peanut und verschiedenen INSV-Isolaten; | | | |
|---|---|---|---|
| Isolat | Infiziert Kontrollpflanzen nach mechanischer Inokulation | Infizierte transgene Pflanzen nach mechanischer Inokulation | Art der Merkmale infizierter Pflanzen |
| TSWV-Peanut = SA05 (DSM No. | 100 % | 4 % | Schwere systemische Infektion |
| PV-0205) INSV = (DSM No. PV-0280) | 95 % | 55 % | Lokalläsionen auf den inokulierten Blättern; Keine systemische Infektion |
| INSV (DSM No. PV-0281) | 85 % | 35 % | Lokalläsionen auf den inokulierten Blättern; Keine systemische Infektion |

Biotest von XL 4.24 transgenen Pflanzen mit Virusisolaten, die nicht zur Familie der Bunyaviren gehören.

(a) Testbeschreibung

Homozygote Nachkommen der transgenen Pflanze XL4 (XL 4.24) wurden so lange herangezogen, bis sie das Vierblattstadium erreicht hatten.

Die Pflanzen wurden in Gruppen von 20 zufällig zusammengefaßt und mechanisch mit Homogenaten aus mit verschiedenen Viren infizierten Tabakpflanzen inokuliert. Folgende Virusisolate wurden für die Inokulation verwendet: TMV (DSM No. PV-0055) und Tobacco Rattle Virus (DSM No. PV-0043). Die Pflanzen zur Gewinnung des Inokulums waren 10-14 Tage vorinokuliert und nach elektronenmikroskopischen Befund frei von Kontaminationen mit anderen Viren. Für die Herstellung des Inokulums wurde der Standard DSM Inokulationspuffer verwendet (DSM-Katalog Pflanzenviren), die Homogenisation erfolgte mit Hilfe von Mörsem und Pistill im Verhältnis 1:30 (g/ml). Das Inokulum wurde mit sterilen Glasspateln auf die mit Carborundum (600 mesh) bestäubten Blätter der Testpflanzen abgerieben. Von jeder Testpflanze wurden die zwei ältesten Folgeblätter inokuliert. Nach der Inokulation wurden die infizierten Blätter mit Leitungswasser abgewaschen. Anschließend wurden die Pflanzen unter den oben angeführten Kulturbedingungen inkubiert und täglich auf Symptome visuell bonitiert. Als Kontrollen wurden nicht transformierte *N. tabacum* Pflanzen in gleicher Weise angezogen und mit den verschiedenen Isolaten inokuliert.

Die endgültigen Bonituren des Versuches erfolgten eine und drei Wochen nach der Inokulation durch zwei Personen, bei ungleicher Bewertung wurde die abwertendere Bewertung herangezogen. Die Infektion mit beiden Viren führte in den Kontrollpflanzen sowohl zu lokalen Nekrosen auf den primär infizierten Blättern, im Fall von TRV auch zu starken systemischen Symptomen. Im Falle von TMV waren in den transgenen Pflanzen XL4.24 signifikant weniger Lokalläsionen zu beobachten. Im Falle des Tabacco rattle Virus zeigten die Kontrollen in 14% der Fälle nur Nekrosen auf den primär infizierten Blättern während 86% auch systemische Symptome zeigten. Dagegen waren 28% der transgenen Pflanzen vollständig befallsfrei, 36% zeigten außschließlich Primärnekrosen und nur 36% hatten auch systemische Symptome.

3.3 Erhöhte Resistenz gegenüber planzenpathogener Nematoden

(a) Testbeschreibung

Die transgenen Tabakpflanzen (XL4) wurden bei 23°C und 70-80% rel. Luftfeuchte im Gewächshaus bis Versuchsbeginn angezogen. Nach dem Pikieren wurden die Pflanzen in Landerde weiter angezogen um ein besonders gut ausgebildetes Wurzelsystem zu gewährleisten. Die Versorgung mit Wasser und Dünger erfolgte nach Bedarf. Zur Inokulation wurden bei Pflanzen, die ca. 15cm hoch waren ca. 300 vitale, frisch geschlüpfte Larven von *Meloidogyne incognita* (LII) pro 100ml Bodenvolumen gleichmäßig auf die Bodenoberfläche pipetiert. Die Bonitur erfolgte 4 Wochen nach Inokulation durch Ermittlung der Gallenzahl jedes Wurzelsystems. Die transgenen Pflanzen weisen im Vergleich zu den Wildtyp-Pflanzen gegenüber Nematoden eine erhöhte Wiederstandsfähigkeit auf.

Im vorliegenden Text beziehen sich %-Angaben auf Gewichtsprozente, wenn nichts anderes angegeben wird und Verdünnungen auf Volumenteile (z.B. 1:25), wenn nichts anderes angegeben wird.

```
                                   SEQUENZPROTOKOLL


     (1)  ALGEMEINE  INFORMATION:

          (i)  ANMELDER:
               (A)  NAME: Bayer AG
               (B)  STRASSE: Bayerwerk
               (C)  ORT: Leverkusen
               (E)  LAND: Deutschland
               (F)  POSTLEITZAHL: 5090
               (G)  TELEPHON: 0214/30 61285
               (H)  TELEFAX: 0214/30 3482
               (I)  TELEX: 85 101-265 by d


          (ii)  ANMELDETITEL: Desoxyribonucleinsauren

          (iii)  ANZAHL DER SEQUENZEN: 18

          (iv)  COMPUTER-LESBARE FORM:
               (A)  DATENTRÄGER: Floppy disk
               (B)  COMPUTER: IBM PC compatible
               (C)  BETRIEBSSYSTEM: PC-DOS/MS-DOS
               (D)  SOFTWARE: PatentIn Release #1.0, Version #1.25 (EPA)


     (2)  INFORMATION ZU SEQ ID NO: 1:

          (i)  SEQUENZ CHARAKTERISTIKA:
               (A)  LÄNGE: 1040 Basenpaare
               (B)  ART: Nukleinsäure
               (C)  STRANGFORM: Einzel
               (D)  TOPOLOGIE: linear

          (ii)  ART DES MOLEKÜLS: DNS (genomisch)


          (ix)  MERKMALE:
               (A)  NAME/SCHLÜSSEL: CDS
               (B)  LAGE: 36..989


          (xi)  SEQUENZBESCHREIBUNG: SEQ ID NO: 1:

     AAAATATAAA AACTCAACAC AACATACAAA ATTTT ATG CAA TCA AAT CAA TCT        53
                                           Met Gln Ser Asn Gln Ser
                                            1               5

     CAA GCT ATC AAA ATT TTT CGA ATC TCA CTT GAA AGA TCA AAA ATC AAC     101
     Gln Ala Ile Lys Ile Phe Arg Ile Ser Leu Glu Arg Ser Lys Ile Asn
                   10              15              20

     AAA GAA AAT TCC TTA ATT TCT TTA CTA AAT TTA CTG CAA GTC AAG ATG     149
     Lys Glu Asn Ser Leu Ile Ser Leu Leu Asn Leu Leu Gln Val Lys Met
                   25              30              35

     TCG AGA ATT CGA GCT CGG TAC CCA CCC GAT CCT CTA GAG TCG CAT ATA     197
     Ser Arg Ile Arg Ala Arg Tyr Pro Pro Asp Pro Leu Glu Ser His Ile
                   40              45              50

     ACA ACT TCT ACA ATC ATC ATG TCT AAG GTT AAG CTC ACT AAG GAA AGC     245
     Thr Thr Ser Thr Ile Ile Met Ser Lys Val Lys Leu Thr Lys Glu Ser
      55              60              65              70
```

```
ATT GTT GCT TTG TTG ACA CAA GGC AAA GAC CTT GAG TTT GAG GAA GAT        293
Ile Val Ala Leu Leu Thr Gln Gly Lys Asp Leu Glu Phe Glu Glu Asp
                    75                  80                  85

CAG AAT CTG GTA GCA TTC AAC TTC AAG ACT TTT TGT CTA GAA AAC CTC        341
Gln Asn Leu Val Ala Phe Asn Phe Lys Thr Phe Cys Leu Glu Asn Leu
                90                  95                 100

GAC CAG ATC AAG AAG ATG AGC GTT ATT TCA TGT CTG ACG TTC CTG AAG        389
Asp Gln Ile Lys Lys Met Ser Val Ile Ser Cys Leu Thr Phe Leu Lys
            105                 110                 115

AAT CGT CAG AGT ATA ATG AAG GTT ATT AAA CAA AGT GAT TTT ACT TTT        437
Asn Arg Gln Ser Ile Met Lys Val Ile Lys Gln Ser Asp Phe Thr Phe
        120                 125                 130

GGT AAA ATT ACC ATA AAG AAA ACT TCA GAC AGG ATT GGA GCC ACT GAC        485
Gly Lys Ile Thr Ile Lys Lys Thr Ser Asp Arg Ile Gly Ala Thr Asp
135                 140                 145                 150

ATG ACC TTC AGA AGG CTT GAT AGC TTG ATC AGG GTC AGG CTT GTA GAG        533
Met Thr Phe Arg Arg Leu Asp Ser Leu Ile Arg Val Arg Leu Val Glu
                155                 160                 165

GAA ACC GGG AAT TCT GAG AAT CTC AAT ACT ATC AAA TCT AAG ATT GCT        581
Glu Thr Gly Asn Ser Glu Asn Leu Asn Thr Ile Lys Ser Lys Ile Ala
                170                 175                 180

TCC CAC CCT TTG ATT CAA GCC TAT GGA TTA CCT CTT GAT GAT GCA AAG        629
Ser His Pro Leu Ile Gln Ala Tyr Gly Leu Pro Leu Asp Asp Ala Lys
            185                 190                 195

TCT GTG AGG CTT GCC ATA ATG CTG GGA GGT AGC TTA CCT CTT ATT GCT        677
Ser Val Arg Leu Ala Ile Met Leu Gly Gly Ser Leu Pro Leu Ile Ala
        200                 205                 210

TCA GTT GAT AGC TTT GAG ATG ATC AGT GTT GTC TTG GCT ATA TAT CAG        725
Ser Val Asp Ser Phe Glu Met Ile Ser Val Val Leu Ala Ile Tyr Gln
215                 220                 225                 230

GAT GCA AAA TAC AAA GAC CTC GGG ATT GAC CCA AAG AAG TAT GAC ACC        773
Asp Ala Lys Tyr Lys Asp Leu Gly Ile Asp Pro Lys Lys Tyr Asp Thr
            235                 240                 245

AAG GAA GCC TTA GGA AAA GTT TGC ACT GTG CTG AAA AGC AAA GCA TTT        821
Lys Glu Ala Leu Gly Lys Val Cys Thr Val Leu Lys Ser Lys Ala Phe
            250                 255                 260

GAA ATG AAT GAA GAT CAG GTG AAG AAA GGA AAA GAG TAT GCT GCT ATA        869
Glu Met Asn Glu Asp Gln Val Lys Lys Gly Lys Glu Tyr Ala Ala Ile
            265                 270                 275

CTC AGC TCC AGC AAT CCT AAT GCT AAA GGA AGT ATT GCT ATG GAA CAT        917
Leu Ser Ser Ser Asn Pro Asn Ala Lys Gly Ser Ile Ala Met Glu His
        280                 285                 290

TAC AGT GAA ACT CTT AAC AAG TTC TAT GAA ATG TTT GGG GTT AAA AAA        965
Tyr Ser Glu Thr Leu Asn Lys Phe Tyr Glu Met Phe Gly Val Lys Lys
295                 300                 305                 310

CAG GCA AAA CTC ACA GAA CTT GCT TAAAAGCAGT TGTAAGTTAA ATTATGGAAA      1019
Gln Ala Lys Leu Thr Glu Leu Ala
                315
```

AGTCTACAAA TATATAAAGC T                                                      1040

(2) INFORMATION ZU SEQ ID NO: 2:

    (i) SEQUENZ CHARAKTERISTIKA:
      (A) LÄNGE: 318 Aminosäuren
      (B) ART: Aminosäure
      (D) TOPOLOGIE: linear

    (ii) ART DES MOLEKÜLS: Protein

    (xi) SEQUENZBESCHREIBUNG: SEQ ID NO: 2:

```
Met Gln Ser Asn Gln Ser Gln Ala Ile Lys Ile Phe Arg Ile Ser Leu
 1               5                  10                  15

Glu Arg Ser Lys Ile Asn Lys Glu Asn Ser Leu Ile Ser Leu Leu Asn
            20                  25                  30

Leu Leu Gln Val Lys Met Ser Arg Ile Arg Ala Arg Tyr Pro Pro Asp
        35                  40                  45

Pro Leu Glu Ser His Ile Thr Thr Ser Thr Ile Ile Met Ser Lys Val
    50                  55                  60

Lys Leu Thr Lys Glu Ser Ile Val Ala Leu Leu Thr Gln Gly Lys Asp
65                  70                  75                  80

Leu Glu Phe Glu Glu Asp Gln Asn Leu Val Ala Phe Asn Phe Lys Thr
                85                  90                  95

Phe Cys Leu Glu Asn Leu Asp Gln Ile Lys Lys Met Ser Val Ile Ser
            100                 105                 110

Cys Leu Thr Phe Leu Lys Asn Arg Gln Ser Ile Met Lys Val Ile Lys
        115                 120                 125

Gln Ser Asp Phe Thr Phe Gly Lys Ile Thr Ile Lys Lys Thr Ser Asp
    130                 135                 140

Arg Ile Gly Ala Thr Asp Met Thr Phe Arg Arg Leu Asp Ser Leu Ile
145                 150                 155                 160

Arg Val Arg Leu Val Glu Glu Thr Gly Asn Ser Glu Asn Leu Asn Thr
                165                 170                 175

Ile Lys Ser Lys Ile Ala Ser His Pro Leu Ile Gln Ala Tyr Gly Leu
            180                 185                 190

Pro Leu Asp Asp Ala Lys Ser Val Arg Leu Ala Ile Met Leu Gly Gly
        195                 200                 205

Ser Leu Pro Leu Ile Ala Ser Val Asp Ser Phe Glu Met Ile Ser Val
    210                 215                 220

Val Leu Ala Ile Tyr Gln Asp Ala Lys Tyr Lys Asp Leu Gly Ile Asp
225                 230                 235                 240

Pro Lys Lys Tyr Asp Thr Lys Glu Ala Leu Gly Lys Val Cys Thr Val
            245                 250                 255

Leu Lys Ser Lys Ala Phe Glu Met Asn Glu Asp Gln Val Lys Lys Gly
            260                 265                 270
```

```
Lys Glu Tyr Ala Ala Ile Leu Ser Ser Ser Asn Pro Asn Ala Lys Gly
        275             280             285

Ser Ile Ala Met Glu His Tyr Ser Glu Thr Leu Asn Lys Phe Tyr Glu
        290                 295             300

Met Phe Gly Val Lys Lys Gln Ala Lys Leu Thr Glu Leu Ala
305             310             315
```

(2)  INFORMATION ZU SEQ ID NO: 3:

    (i)  SEQUENZ CHARAKTERISTIKA:
        (A)  LÄNGE: 439 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)

    (xi)  SEQUENZBESCHREIBUNG: SEQ ID NO: 3:

```
AAGCTTGCAT GCCTGCAGGT CAACATGGTG GAGCACGACA CTCTCGTCTA CTCCAAGAAT        60

ATCAAAGATA CAGTCTCAGA AGACCAAAGG GCTATTGAGA CTTTTCAACA AAGGGTAATA       120

TCGGGAAACC TCCTCGGATT CCATTGCCCA GCTATCTGTC ACTTGATCAA AAGGACAGTA       180

GAAAAGGAAG GTGGCACCTA CAAATGCCAT CATTGCGATA AAGGAAAGGC TATCGTTCAA       240

GAATGCCTCT GCCGACAGTG GTCCCAAAGA TGGACCCCCA CCCACGAGGA GCATCGTGGA       300

AAAAGAAGAC GTTCCAACCA CGTCTTCAAA GCAAGTGGAT TGATGTGATA TCTCCACTGA       360

CGTAAGGGAT GACGCACAAT CCCACTATCC TTCGCAAGAC CCTTCCTCTA TATAAGGAAG       420

TTCATTTCAT TTGGAGAGG                                                    439
```

(2)  INFORMATION ZU SEQ ID NO: 4:

    (i)  SEQUENZ CHARAKTERISTIKA:
        (A)  LÄNGE: 226 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)

    (xi)  SEQUENZBESCHREIBUNG: SEQ ID NO: 4:

```
CGCAAAAATC ACCAGTCTCT CTCTACAAAT CTATCTCTCT CTATTTTTCT CCAGAATAAT        60

GTGTGAGTAG TTCCCAGATA AGGGAATTAG GGTTCTTATA GGGTTTCGCT CATGTGTTGA       120

GCATATAAGA AACCCTTAGT ATGTATTTGT ATTTGTAAAA TACTTCTATC AATAAAATTT       180

CTAATTCCTA AAACCAAAAT CCAGTGACCT GCAGGCATGC AAGCTT                      226
```

(2) INFORMATION ZU SEQ ID NO: 5:

    (i) SEQUENZ CHARAKTERISTIKA:
      (A) LÄNGE: 1722 Basenpaare
      (B) ART: Nukleinsäure
      (C) STRANGFORM: Einzel
      (D) TOPOLOGIE: linear

    (ii) ART DES MOLEKÜLS: DNS (genomisch)

    (ix) MERKMALE:
      (A) NAME/SCHLÜSSEL: CDS
      (B) LAGE: 475..1428

    (xi) SEQUENZBESCHREIBUNG: SEQ ID NO: 5:

```
AAGCTTGCAT GCCTGCAGGT CAACATGGTG GAGCACGACA CTCTCGTCTA CTCCAAGAAT          60

ATCAAAGATA CAGTCTCAGA AGACCAAAGG GCTATTGAGA CTTTTCAACA AAGGGTAATA         120

TCGGGAAACC TCCTCGGATT CCATTGCCCA GCTATCTGTC ACTTGATCAA AAGGACAGTA         180

GAAAAGGAAG GTGGCACCTA CAAATGCCAT CATTGCGATA AAGGAAAGGC TATCGTTCAA         240

GAATGCCTCT GCCGACAGTG GTCCCAAAGA TGGACCCCCA CCCACGAGGA GCATCGTGGA         300

AAAAGAAGAC GTTCCAACCA CGTCTTCAAA GCAAGTGGAT TGATGTGATA TCTCCACTGA         360

CGTAAGGGAT GACGCACAAT CCCACTATCC TTCGCAAGAC CCTTCCTCTA TATAAGGAAG         420

TTCATTTCAT TTGGAGAGGA AAATATAAAA ACTCAACACA ACATACAAAA TTTT ATG          477
                                                                   Met
                                                                     1

CAA TCA AAT CAA TCT CAA GCT ATC AAA ATT TTT CGA ATC TCA CTT GAA          525
Gln Ser Asn Gln Ser Gln Ala Ile Lys Ile Phe Arg Ile Ser Leu Glu
              5                  10                  15

AGA TCA AAA ATC AAC AAA GAA AAT TCC TTA ATT TCT TTA CTA AAT TTA          573
Arg Ser Lys Ile Asn Lys Glu Asn Ser Leu Ile Ser Leu Leu Asn Leu
           20                  25                  30

CTG CAA GTC AAG ATG TCG AGA ATT CGA GCT CGG TAC CCA CCC GAT CCT          621
Leu Gln Val Lys Met Ser Arg Ile Arg Ala Arg Tyr Pro Pro Asp Pro
        35                  40                  45

CTA GAG TCG CAT ATA ACA ACT TCT ACA ATC ATC ATG TCT AAG GTT AAG          669
Leu Glu Ser His Ile Thr Thr Ser Thr Ile Ile Met Ser Lys Val Lys
  50                  55                  60                  65

CTC ACT AAG GAA AGC ATT GTT GCT TTG TTG ACA CAA GGC AAA GAC CTT          717
Leu Thr Lys Glu Ser Ile Val Ala Leu Leu Thr Gln Gly Lys Asp Leu
                 70                  75                  80

GAG TTT GAG GAA GAT CAG AAT CTG GTA GCA TTC AAC TTC AAG ACT TTT          765
Glu Phe Glu Glu Asp Gln Asn Leu Val Ala Phe Asn Phe Lys Thr Phe
                 85                  90                  95

TGT CTA GAA AAC CTC GAC CAG ATC AAG AAG ATG AGC GTT ATT TCA TGT          813
Cys Leu Glu Asn Leu Asp Gln Ile Lys Lys Met Ser Val Ile Ser Cys
           100                 105                 110
```

```
CTG ACG TTC CTG AAG AAT CGT CAG AGT ATA ATG AAG GTT ATT AAA CAA          861
Leu Thr Phe Leu Lys Asn Arg Gln Ser Ile Met Lys Val Ile Lys Gln
    115                 120                 125

AGT GAT TTT ACT TTT GGT AAA ATT ACC ATA AAG AAA ACT TCA GAC AGG          909
Ser Asp Phe Thr Phe Gly Lys Ile Thr Ile Lys Lys Thr Ser Asp Arg
130                 135                 140                 145

ATT GGA GCC ACT GAC ATG ACC TTC AGA AGG CTT GAT AGC TTG ATC AGG          957
Ile Gly Ala Thr Asp Met Thr Phe Arg Arg Leu Asp Ser Leu Ile Arg
                150                 155                 160

GTC AGG CTT GTA GAG GAA ACC GGG AAT TCT GAG AAT CTC AAT ACT ATC         1005
Val Arg Leu Val Glu Glu Thr Gly Asn Ser Glu Asn Leu Asn Thr Ile
                165                 170                 175

AAA TCT AAG ATT GCT TCC CAC CCT TTG ATT CAA GCC TAT GGA TTA CCT         1053
Lys Ser Lys Ile Ala Ser His Pro Leu Ile Gln Ala Tyr Gly Leu Pro
                180                 185                 190

CTT GAT GAT GCA AAG TCT GTG AGG CTT GCC ATA ATG CTG GGA GGT AGC         1101
Leu Asp Asp Ala Lys Ser Val Arg Leu Ala Ile Met Leu Gly Gly Ser
                195                 200                 205

TTA CCT CTT ATT GCT TCA GTT GAT AGC TTT GAG ATG ATC AGT GTT GTC         1149
Leu Pro Leu Ile Ala Ser Val Asp Ser Phe Glu Met Ile Ser Val Val
210                 215                 220                 225

TTG GCT ATA TAT CAG GAT GCA AAA TAC AAA GAC CTC GGG ATT GAC CCA         1197
Leu Ala Ile Tyr Gln Asp Ala Lys Tyr Lys Asp Leu Gly Ile Asp Pro
                230                 235                 240

AAG AAG TAT GAC ACC AAG GAA GCC TTA GGA AAA GTT TGC ACT GTG CTG         1245
Lys Lys Tyr Asp Thr Lys Glu Ala Leu Gly Lys Val Cys Thr Val Leu
                245                 250                 255

AAA AGC AAA GCA TTT GAA ATG AAT GAA GAT CAG GTG AAG AAA GGA AAA         1293
Lys Ser Lys Ala Phe Glu Met Asn Glu Asp Gln Val Lys Lys Gly Lys
                260                 265                 270

GAG TAT GCT GCT ATA CTC AGC TCC AGC AAT CCT AAT GCT AAA GGA AGT         1341
Glu Tyr Ala Ala Ile Leu Ser Ser Ser Asn Pro Asn Ala Lys Gly Ser
    275                 280                 285

ATT GCT ATG GAA CAT TAC AGT GAA ACT CTT AAC AAG TTC TAT GAA ATG         1389
Ile Ala Met Glu His Tyr Ser Glu Thr Leu Asn Lys Phe Tyr Glu Met
290                 295                 300                 305

TTT GGG GTT AAA AAA CAG GCA AAA CTC ACA GAA CTT GCT TAAAAGCAGT          1438
Phe Gly Val Lys Lys Gln Ala Lys Leu Thr Glu Leu Ala
                310                 315

TGTAAGTTAA ATTATGGAAA AGTCTACAAA TATATAAAGC TGGGGATCCT CTAGAGTCCG       1498

CAAAAATCAC CAGTCTCTCT CTACAAATCT ATCTCTCTCT ATTTTTCTCC AGAATAATGT       1558

GTGAGTAGTT CCCAGATAAG GGAATTAGGG TTCTTATAGG GTTTCGCTCA TGTGTTGAGC       1618

ATATAAGAAA CCCTTAGTAT GTATTTGTAT TTGTAAAATA CTTCTATCAA TAAAATTTCT       1678

AATTCCTAAA ACCAAAATCC AGTGACCTGC AGGCATGCAA GCTT                        1722
```

(2) INFORMATION ZU SEQ ID NO: 6:

    (i) SEQUENZ CHARAKTERISTIKA:
       (A) LÄNGE: 318 Aminosäuren
       (B) ART: Aminosäure
       (D) TOPOLOGIE: linear

   (ii) ART DES MOLEKÜLS: Protein

   (xi) SEQUENZBESCHREIBUNG: SEQ ID NO: 6:

```
Met Gln Ser Asn Gln Ser Gln Ala Ile Lys Ile Phe Arg Ile Ser Leu
 1               5                  10                  15

Glu Arg Ser Lys Ile Asn Lys Glu Asn Ser Leu Ile Ser Leu Leu Asn
            20                  25                  30

Leu Leu Gln Val Lys Met Ser Arg Ile Arg Ala Arg Tyr Pro Pro Asp
        35                  40                  45

Pro Leu Glu Ser His Ile Thr Thr Ser Thr Ile Ile Met Ser Lys Val
    50                  55                  60

Lys Leu Thr Lys Glu Ser Ile Val Ala Leu Leu Thr Gln Gly Lys Asp
65                  70                  75                  80

Leu Glu Phe Glu Glu Asp Gln Asn Leu Val Ala Phe Asn Phe Lys Thr
            85                  90                  95

Phe Cys Leu Glu Asn Leu Asp Gln Ile Lys Lys Met Ser Val Ile Ser
            100                 105                 110

Cys Leu Thr Phe Leu Lys Asn Arg Gln Ser Ile Met Lys Val Ile Lys
        115                 120                 125

Gln Ser Asp Phe Thr Phe Gly Lys Ile Thr Ile Lys Lys Thr Ser Asp
        130                 135                 140

Arg Ile Gly Ala Thr Asp Met Thr Phe Arg Arg Leu Asp Ser Leu Ile
145                 150                 155                 160

Arg Val Arg Leu Val Glu Glu Thr Gly Asn Ser Glu Asn Leu Asn Thr
            165                 170                 175

Ile Lys Ser Lys Ile Ala Ser His Pro Leu Ile Gln Ala Tyr Gly Leu
            180                 185                 190

Pro Leu Asp Asp Ala Lys Ser Val Arg Leu Ala Ile Met Leu Gly Gly
        195                 200                 205

Ser Leu Pro Leu Ile Ala Ser Val Asp Ser Phe Glu Met Ile Ser Val
        210                 215                 220

Val Leu Ala Ile Tyr Gln Asp Ala Lys Tyr Lys Asp Leu Gly Ile Asp
225                 230                 235                 240

Pro Lys Lys Tyr Asp Thr Lys Glu Ala Leu Gly Lys Val Cys Thr Val
            245                 250                 255

Leu Lys Ser Lys Ala Phe Glu Met Asn Glu Asp Gln Val Lys Lys Gly
            260                 265                 270

Lys Glu Tyr Ala Ala Ile Leu Ser Ser Ser Asn Pro Asn Ala Lys Gly
            275                 280                 285
```

20

```
Ser Ile Ala Met Glu His Tyr Ser Glu Thr Leu Asn Lys Phe Tyr Glu
    290                 295             300

Met Phe Gly Val Lys Lys Gln Ala Lys Leu Thr Glu Leu Ala
    305                 310             315
```

(2) INFORMATION ZU SEQ ID NO: 7:

    (i) SEQUENZ CHARAKTERISTIKA:
        (A) LÄNGE: 152 Basenpaare
        (B) ART: Nukleinsäure
        (C) STRANGFORM: Einzel
        (D) TOPOLOGIE: linear

    (ii) ART DES MOLEKÜLS: DNS (genomisch)

    (ix) MERKMALE:
        (A) NAME/SCHLÜSSEL: CDS
        (B) LAGE: 36..152

    (xi) SEQUENZBESCHREIBUNG: SEQ ID NO: 7:

```
AAAATATAAA AACTCAACAC AACATACAAA ATTTT ATG CAA TCA AAT CAA TCT      53
                                        Met Gln Ser Asn Gln Ser
                                          1                 5

CAA GCT ATC AAA ATT TTT CGA ATC TCA CTT GAA AGA TCA AAA ATC AAC    101
Gln Ala Ile Lys Ile Phe Arg Ile Ser Leu Glu Arg Ser Lys Ile Asn
            10              15              20

AAA GAA AAT TCC TTA ATT TCT TTA CTA AAT TTA CTG CAA GTC AAG ATG    149
Lys Glu Asn Ser Leu Ile Ser Leu Leu Asn Leu Leu Gln Val Lys Met
        25              30              35

TCG                                                                152
Ser
```

(2) INFORMATION ZU SEQ ID NO: 8:

    (i) SEQUENZ CHARAKTERISTIKA:
        (A) LÄNGE: 39 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 8:

```
Met Gln Ser Asn Gln Ser Gln Ala Ile Lys Ile Phe Arg Ile Ser Leu
  1                 5                 10              15

Glu Arg Ser Lys Ile Asn Lys Glu Asn Ser Leu Ile Ser Leu Leu Asn
            20              25              30

Leu Leu Gln Val Lys Met Ser
        35
```

21

(2)  INFORMATION ZU SEQ ID NO: 9:

    (i)  SEQUENZ CHARAKTERISTIKA:
       (A)  LÄNGE: 38 Basenpaare
       (B)  ART: Nukleinsäure
       (C)  STRANGFORM: Einzel
       (D)  TOPOLOGIE: linear

    (ii) ART DES MOLEKÜLS: DNS (genomisch)

    (ix) MERKMALE:
       (A)  NAME/SCHLÜSSEL: CDS
       (B)  LAGE: 1..36

    (xi) SEQUENZBESCHREIBUNG: SEQ ID NO: 9:

```
AGA ATT CGA GCT CGG TAC CCA CCC GAT CCT CTA GAG TC        38
Arg Ile Arg Ala Arg Tyr Pro Pro Asp Pro Leu Glu
 1           5                   10
```

(2)  INFORMATION ZU SEQ ID NO: 10:

    (i)  SEQUENZ CHARAKTERISTIKA:
       (A)  LÄNGE: 12 Aminosäuren
       (B)  ART: Aminosäure
       (D)  TOPOLOGIE: linear

    (ii) ART DES MOLEKÜLS: Protein

    (xi) SEQUENZBESCHREIBUNG: SEQ ID NO: 10:

```
Arg Ile Arg Ala Arg Tyr Pro Pro Asp Pro Leu Glu
 1           5                   10
```

(2)  INFORMATION ZU SEQ ID NO: 11:

    (i)  SEQUENZ CHARAKTERISTIKA:
       (A)  LÄNGE: 850 Basenpaare
       (B)  ART: Nukleinsäure
       (C)  STRANGFORM: Einzel
       (D)  TOPOLOGIE: linear

    (ii) ART DES MOLEKÜLS: DNS (genomisch)

    (ix) MERKMALE:
       (A)  NAME/SCHLÜSSEL: CDS
       (B)  LAGE: 2..799

    (xi) SEQUENZBESCHREIBUNG: SEQ ID NO: 11:

```
G CAT ATA ACA ACT TCT ACA ATC ATC ATG TCT AAG GTT AAG CTC ACT        46
  His Ile Thr Thr Ser Thr Ile Ile Met Ser Lys Val Lys Leu Thr
   1           5                   10                  15

AAG GAA AGC ATT GTT GCT TTG TTG ACA CAA GGC AAA GAC CTT GAG TTT        94
Lys Glu Ser Ile Val Ala Leu Leu Thr Gln Gly Lys Asp Leu Glu Phe
           20                  25                  30
```

```
GAG GAA GAT CAG AAT CTG GTA GCA TTC AAC TTC AAG ACT TTT TGT CTA        142
Glu Glu Asp Gln Asn Leu Val Ala Phe Asn Phe Lys Thr Phe Cys Leu
            35              40              45

GAA AAC CTC GAC CAG ATC AAG AAG ATG AGC GTT ATT TCA TGT CTG ACG        190
Glu Asn Leu Asp Gln Ile Lys Lys Met Ser Val Ile Ser Cys Leu Thr
            50              55              60

TTC CTG AAG AAT CGT CAG AGT ATA ATG AAG GTT ATT AAA CAA AGT GAT        238
Phe Leu Lys Asn Arg Gln Ser Ile Met Lys Val Ile Lys Gln Ser Asp
        65              70              75

TTT ACT TTT GGT AAA ATT ACC ATA AAG AAA ACT TCA GAC AGG ATT GGA        286
Phe Thr Phe Gly Lys Ile Thr Ile Lys Lys Thr Ser Asp Arg Ile Gly
    80              85              90              95

GCC ACT GAC ATG ACC TTC AGA AGG CTT GAT AGC TTG ATC AGG GTC AGG        334
Ala Thr Asp Met Thr Phe Arg Arg Leu Asp Ser Leu Ile Arg Val Arg
            100             105             110

CTT GTA GAG GAA ACC GGG AAT TCT GAG AAT CTC AAT ACT ATC AAA TCT        382
Leu Val Glu Glu Thr Gly Asn Ser Glu Asn Leu Asn Thr Ile Lys Ser
            115             120             125

AAG ATT GCT TCC CAC CCT TTG ATT CAA GCC TAT GGA TTA CCT CTT GAT        430
Lys Ile Ala Ser His Pro Leu Ile Gln Ala Tyr Gly Leu Pro Leu Asp
            130             135             140

GAT GCA AAG TCT GTG AGG CTT GCC ATA ATG CTG GGA GGT AGC TTA CCT        478
Asp Ala Lys Ser Val Arg Leu Ala Ile Met Leu Gly Gly Ser Leu Pro
            145             150             155

CTT ATT GCT TCA GTT GAT AGC TTT GAG ATG ATC AGT GTT GTC TTG GCT        526
Leu Ile Ala Ser Val Asp Ser Phe Glu Met Ile Ser Val Val Leu Ala
160             165             170             175

ATA TAT CAG GAT GCA AAA TAC AAA GAC CTC GGG ATT GAC CCA AAG AAG        574
Ile Tyr Gln Asp Ala Lys Tyr Lys Asp Leu Gly Ile Asp Pro Lys Lys
            180             185             190

TAT GAC ACC AAG GAA GCC TTA GGA AAA GTT TGC ACT GTG CTG AAA AGC        622
Tyr Asp Thr Lys Glu Ala Leu Gly Lys Val Cys Thr Val Leu Lys Ser
            195             200             205

AAA GCA TTT GAA ATG AAT GAA GAT CAG GTG AAG AAA GGA AAA GAG TAT        670
Lys Ala Phe Glu Met Asn Glu Asp Gln Val Lys Lys Gly Lys Glu Tyr
            210             215             220

GCT GCT ATA CTC AGC TCC AGC AAT CCT AAT GCT AAA GGA AGT ATT GCT        718
Ala Ala Ile Leu Ser Ser Ser Asn Pro Asn Ala Lys Gly Ser Ile Ala
            225             230             235

ATG GAA CAT TAC AGT GAA ACT CTT AAC AAG TTC TAT GAA ATG TTT GGG        766
Met Glu His Tyr Ser Glu Thr Leu Asn Lys Phe Tyr Glu Met Phe Gly
240             245             250             255

GTT AAA AAA CAG GCA AAA CTC ACA GAA CTT GCT TAAAAGCAGT TGTAAGTTAA      819
Val Lys Lys Gln Ala Lys Leu Thr Glu Leu Ala
            260             265

ATTATGGAAA AGTCTACAAA TATATAAAGC T                                     850
```

(2) INFORMATION ZU SEQ ID NO: 12:

    (i) SEQUENZ CHARAKTERISTIKA:
       (A) LÄNGE: 266 Aminosäuren
       (B) ART: Aminosäure
       (D) TOPOLOGIE: linear

    (ii) ART DES MOLEKÜLS: Protein

    (xi) SEQUENZBESCHREIBUNG: SEQ ID NO: 12:

```
His Ile Thr Thr Ser Thr Ile Ile Met Ser Lys Val Lys Leu Thr Lys
 1               5                  10               15

Glu Ser Ile Val Ala Leu Leu Thr Gln Gly Lys Asp Leu Glu Phe Glu
            20                  25                  30

Glu Asp Gln Asn Leu Val Ala Phe Asn Phe Lys Thr Phe Cys Leu Glu
            35                  40                  45

Asn Leu Asp Gln Ile Lys Lys Met Ser Val Ile Ser Cys Leu Thr Phe
            50                  55                  60

Leu Lys Asn Arg Gln Ser Ile Met Lys Val Ile Lys Gln Ser Asp Phe
65                  70                  75                  80

Thr Phe Gly Lys Ile Thr Ile Lys Lys Thr Ser Asp Arg Ile Gly Ala
                85                  90                  95

Thr Asp Met Thr Phe Arg Arg Leu Asp Ser Leu Ile Arg Val Arg Leu
            100                 105                 110

Val Glu Glu Thr Gly Asn Ser Glu Asn Leu Asn Thr Ile Lys Ser Lys
            115                 120                 125

Ile Ala Ser His Pro Leu Ile Gln Ala Tyr Gly Leu Pro Leu Asp Asp
            130                 135                 140

Ala Lys Ser Val Arg Leu Ala Ile Met Leu Gly Gly Ser Leu Pro Leu
145                 150                 155                 160

Ile Ala Ser Val Asp Ser Phe Glu Met Ile Ser Val Val Leu Ala Ile
                165                 170                 175

Tyr Gln Asp Ala Lys Tyr Lys Asp Leu Gly Ile Asp Pro Lys Lys Tyr
            180                 185                 190

Asp Thr Lys Glu Ala Leu Gly Lys Val Cys Thr Val Leu Lys Ser Lys
            195                 200                 205

Ala Phe Glu Met Asn Glu Asp Gln Val Lys Lys Gly Lys Glu Tyr Ala
            210                 215                 220

Ala Ile Leu Ser Ser Ser Asn Pro Asn Ala Lys Gly Ser Ile Ala Met
225                 230                 235                 240

Glu His Tyr Ser Glu Thr Leu Asn Lys Phe Tyr Glu Met Phe Gly Val
            245                 250                 255

Lys Lys Gln Ala Lys Leu Thr Glu Leu Ala
            260                 265
```

24

(2) INFORMATION ZU SEQ ID NO: 13:

    (i) SEQUENZ CHARAKTERISTIKA:
        (A) LÄNGE: 17 Basenpaare
        (B) ART: Nukleinsäure
        (C) STRANGFORM: Einzel
        (D) TOPOLOGIE: linear

    (ii) ART DES MOLEKÜLS: DNS (genomisch)

    (xi) SEQUENZBESCHREIBUNG: SEQ ID NO: 13:

GGGGATCCTC TAGAGTC                    17

(2) INFORMATION ZU SEQ ID NO: 14:

    (i) SEQUENZ CHARAKTERISTIKA:
        (A) LÄNGE: 36 Basenpaare
        (B) ART: Nukleinsäure
        (C) STRANGFORM: Einzel
        (D) TOPOLOGIE: linear

    (ii) ART DES MOLEKÜLS: DNS (genomisch)

    (xi) SEQUENZBESCHREIBUNG: SEQ ID NO: 14:

TGTGTTGAGT TTTTATATTT TCCTCTCCAA ATGAAA        36

(2) INFORMATION ZU SEQ ID NO: 15:

    (i) SEQUENZ CHARAKTERISTIKA:
        (A) LÄNGE: 5 Basenpaare
        (B) ART: Nukleinsäure
        (C) STRANGFORM: Einzel
        (D) TOPOLOGIE: linear

    (ii) ART DES MOLEKÜLS: DNS (genomisch)

    (xi) SEQUENZBESCHREIBUNG: SEQ ID NO: 15:

AGAGG                            5

(2) INFORMATION ZU SEQ ID NO: 16:

    (i) SEQUENZ CHARAKTERISTIKA:
        (A) LÄNGE: 7 Basenpaare
        (B) ART: Nukleinsäure
        (C) STRANGFORM: Einzel
        (D) TOPOLOGIE: linear

    (ii) ART DES MOLEKÜLS: DNS (genomisch)

    (xi) SEQUENZBESCHREIBUNG: SEQ ID NO: 16:

AAAATAT                        7

```
(2)  INFORMATION ZU SEQ ID NO: 17:

        (i)  SEQUENZ CHARAKTERISTIKA:
             (A)  LÄNGE: 26 Basenpaare
             (B)  ART: Nukleinsäure
             (C)  STRANGFORM: Einzel
             (D)  TOPOLOGIE: linear

        (ii)  ART DES MOLEKÜLS: DNS (genomisch)


        (xi)  SEQUENZBESCHREIBUNG: SEQ ID NO: 17:

    GTCAGTGGCT CCAATCCTGT CTGAAG                                    26

(2)  INFORMATION ZU SEQ ID NO: 18:

        (i)  SEQUENZ CHARAKTERISTIKA:
             (A)  LÄNGE: 23 Basenpaare
             (B)  ART: Nukleinsäure
             (C)  STRANGFORM: Einzel
             (D)  TOPOLOGIE: linear

        (ii)  ART DES MOLEKÜLS: DNS (genomisch)


        (xi)  SEQUENZBESCHREIBUNG: SEQ ID NO: 18:

    TGTCGAGAAT TCGAGCTCGG TAC                                       23
```

## Patentansprüche

1. Rekombinante Desoxyribonukleinsäure (DNA), welche dadurch gekennzeichnet ist, daß sie aus einer Kombination der folgenden Bestandteile bestehen oder diese enthalten:
   (a) einem doppelsträngigen cDNA-Fragment das von der RNA des Scharkavirus abgeleitet ist ("Fragment A") und
   (b) einem doppelsträngigen cDNA-Fragment das von der S-RNA des N-Strukturgens des Tomatenbronzefleckenvirus (tomato spotted wilt virus, TSWV) abgeleitet ist ("Fragment B), wobei diese Kombination, neben gegebenenfalls vorhandenen weiteren DNA-Fragmenten, auch einen in Pflanzenzellen wirksamen Promotor aufweisen kann, der die Transkription der rekombinanten DNA-Fragmente regelt.

2. DNA gemäß Anspruch 1, wobei Fragment B hinter Fragment A (also in 3'-Richtung liegt).

3. DNA gemäß Anspruch 1, wobei Fragment A eine DNA mit der in SEQ ID NO:7 aufgeführten Sequenz oder eine gleichwirkende DNA ist.

4. DNA gemäß Anspruch 1, wobei Fragment B eine DNA mit der in SEQ ID NO:11 aufgeführten Sequenz oder eine gleichwirkende DNA ist.

5. DNA gemäß Anspruch 1, welche eine Kombination der DNA-Fragmente, die in SEQ ID NO:7 und SEQ ID NO:11 aufgeführt sind oder eine gleichwirkende DNA Kombination enthält oder aus einer solchen Kombination besteht.

6. DNA gemäß Anspruch 1, welche einen in Pflanzen wirksamen Promotor enthält.

7. DNA gemäß Anspruch 1, welche als Promotor eine DNA mit der CaMV 35S-Promotorsequenz oder eine gleichwirkende DNA enthält.

8. DNA gemäß Anspruch 1, in welcher die Fragmente A und B über ein synthetisches cDNA-Fragment ("Fragment C") mit bis zu 134 Basenpaaren verbunden sind, welches das in Fragment A beginnende offene Leseraster mit dem Fragment B verbindet.

9. DNA gemäß Anspruch 1, in welcher die cDNA-Fragmente A und B über das synthetisches DNA-Fragment mit der Sequenz gemäß SEQ ID NO:9 oder über eine gleichwirkende DNA verbunden sind.

10. DNA gemäß Anspruch 1, die im Anschluß an das Fragment-B ein doppelsträngiges DNA-Fragment ("Fragment D") mit 1 bis 30 Basenpaaren oder eine gleichwirkende DNA enthält.

11. DNA gemäß Anspruch 1, in welcher im Anschluß an das Fragment-B ein doppelsträngiges DNA-Fragment gemäß SEQ ID NO:13 oder eine gleichwirkende DNA enthalten ist.

12. DNA gemäß Anspruch 1, welche eine 3'-untranslatierte DNA enthält, die sich direkt an das Fragment B anschließt oder über ein doppelsträngiges DNA-Fragment (Fragment D) mit Fragment B verbunden ist.

13. DNA gemaß Anspruch 1, welche eine 3'-untranslatierte DNA enthält, die sich direkt an das Fragment B anschließt oder über ein doppelsträngiges DNA-Fragment (Fragment D) mit Fragment B verbunden ist, wobei die 3'-untranslatierte DNA die Sequenz gemäß SEQ ID NO:4 aufweist.

14. DNA gemäß Anspruch 1, welche aus einer DNA mit der in SEQ ID NO:1 aufgeführten Sequenz oder einer gleichwirkenden DNA besteht oder diese enthält.

15. DNA gemäß Anspruch 1, welche aus einer DNA mit der in SEQ ID NO:5 aufgeführten Sequenz oder einer gleichwirkenden DNA besteht oder diese enthält.

16. Virale und prokariotische DNA, die eine DNA nach Anspruch 1 enthält.

17. Pflanzliche DNA, die eine DNA nach Anspruch 1 enthält.

18. Vektoren, enthaltend eine DNA gemäß Anspruch 1.

19. Wirtsorganismen, enthaltend eine DNA gemäß Anspruch 1.

20. Verwendung der rekombinaten DNA gemäß Anspruch 1 zur Erzeugung von Pflanzen, Pflanzenteilen und Pflanzenzellen (einschließlich Vermehrungsmaterial) mit erhöhter Resistenz gegen Schadorganismen oder Pflanzenkrankheiten.

21. Verfahren zur Herstellung von transgenen Pflanzenzellen und Pflanzen, dadurch gekennzeichnet, daß man die rekombinante DNA gemäß Anspruch 1 nach üblichen Methoden in das Genom der Pflanzenzellen oder Pflanzen einsetzt, gegebenenfalls Pflanzenzellen zu Pflanzen regeneriert, gegebenenfalls die Pflanzen vermehrt und gegebenenfalls zur Erzeugung weiterer Pflanzen das Genom der transgenen Pflanzen mit dem Genom weitere Pflanzen kombiniert und die Nachkommen vermehrt.

22. Transgene Pflanzenzellen, Pflanzen und deren Teile sowie Vermehrungsmaterial, welche die rekombinante DNA gemäß Anspruch 1 enthält.

23. Transgene Pflanzenzellen, Pflanzen und deren Teile sowie Vermehrungsmaterial, welche ein Protein gemäß SEQ ID NO:2 bzw. 6 enthalten.